(19) Europäisches Patentamt
European Patent Office
Office européen des brevets

(11) Numéro de publication : **0 457 688 B1**

# (12) FASCICULE DE BREVET EUROPEEN

(45) Date de publication du fascicule du brevet : **27.10.93 Bulletin 93/43**

(51) Int. Cl.$^5$ : **A61K 7/06,** A61K 7/48, A61K 7/08, C11D 1/66, C11D 1/72, C11D 1/755

(21) Numéro de dépôt : **91401276.0**

(22) Date de dépôt : **17.05.91**

## (54) Compositions de lavage à base de silicone et d'alcools gras à groupements éther et/ou thioéther ou sulfoxyde.

(30) Priorité : **18.05.90 FR 9006279**

(43) Date de publication de la demande : **21.11.91 Bulletin 91/47**

(45) Mention de la délivrance du brevet : **27.10.93 Bulletin 93/43**

(84) Etats contractants désignés : **AT BE CH DE DK ES FR GB GR IT LI NL SE**

(56) Documents cités :
**EP-A- 0 373 661**
**DE-A- 2 851 832**
**GB-A- 2 059 959**
**GB-A- 2 060 665**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**
Inventeur : **Dubief, Claude**
**9, rue Edmond Rostand**
**F-78150 Le Chesnay (FR)**
Inventeur : **Beauquey, Bernard**
**40, rue Gaston Paymal**
**F-92110 Clichy (FR)**

(74) Mandataire : **Casalonga, Axel et al**
**BUREAU D.A. CASALONGA - JOSSE**
**Morassistrasse 8**
**D-80469 München (DE)**

**EP 0 457 688 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à des compositions de lavage et de conditionnement des matières kératiniques en particulier des cheveux et/ou de la peau à base de silicone, d'agents tensio-actifs et d'un alcool gras à groupement éther et/ou thioéther ou sulfoxyde ainsi qu'aux procédés de lavage mettant en oeuvre ces compositions.

Les compositions de lavage des matières kératiniques, notamment les shampooings, sont bien connues dans l'état de la technique. On a déjà proposé dans le passé d'utiliser dans de telles compositions, des silicones afin de conférer aux matières traitées, notamment aux cheveux, de bonnes propriétés de conditionnement telles que la douceur, la brillance et la facilité de démêlage.

Compte tenu du caractère insoluble des silicones utilisables dans les compositions de lavage et de conditionnement, on cherche à maintenir les silicones en dispersion régulière dans le milieu sans cependant faire chuter la viscosité et les propriétés détergentes et moussantes des compositions. Les silicones doivent également être véhiculées sur les matières kératiniques traitées en vue de leur conférer, suivant l'application, des propriétés de douceur, de brillance et de démêlage.

La demanderesse a découvert, ce qui fait l'objet de l'invention, qu'en utilisant dans des compositions de lavage à base de silicones insolubles et d'agents tensio-actifs, au moins un alcool ayant 27 à 44 atomes de carbone et comportant un ou deux groupement(s) éther et/ou thioéther ou sulfoxyde, il était possible de préparer des compositions présentant une très bonne homogénéité et une stabilité améliorée, ainsi qu'une viscosité satisfaisante pour l'application sur les matières kératiniques.

Les compositions ainsi préparées possèdent de bonnes propriétés détergentes et moussantes et confèrent aux matières kératiniques notamment aux cheveux et/ou à la peau une grande douceur.

Ces compositions, lorsqu'elles sont appliquées sur les cheveux, possèdent, en plus de leurs propriétés lavantes, des propriétés de conditionnement des cheveux c'est-à-dire que les cheveux traités sont brillants, se démêlent facilement et sont doux au toucher.

L'invention a donc pour objet de nouvelles compositions de lavage à base de silicone, d'agents tensio-actifs et d'alcools éther et/ou thioéther ou sulfoxyde.

Un autre objet de l'invention est constitué par le procédé de lavage mettant en oeuvre de telles compositions.

D'autres objets de l'invention apparaîtront à la lecture de la description et des exemples qui suivent.

Les compositions de lavage des matières kératiniques en particulier des cheveux et de la peau conformes à l'invention comprennent dans un milieu aqueux au moins une silicone, un agent tensio-actif possédant des propriétés détergentes et au moins un alcool ayant 27 à 44 atomes de carbone et comportant un ou deux groupement(s) éther et/ou thioéther ou sulfoxyde répondant à la formule (I)

$$R_1 - X \left[ C_2H_3(OH) \right] CH_2 - Y - R_2 \qquad (I)$$

dans laquelle $R_1$ et $R_2$ désignent, indépendamment l'un de l'autre, des groupements alkyle linéaires en $C_{12}$ à $C_{20}$;

X désigne un atome d'oxygène, un atome de soufre, un groupement sulfoxyde;

Y désigne un atome d'oxygène, de soufre, un groupement sulfoxyde ou méthylène;

dans le cas où Y désigne un groupement méthylène la somme du nombre d'atomes de carbone présents dans les groupements $R_1$ et $R_2$ a une valeur variant de 24 à 40 et de préférence de 26 à 36, inclus;

lorsque Y ne désigne pas un groupement méthylène, la somme des atomes de carbone de $R_1$ et $R_2$ a une valeur variant de 24 à 40 inclus et de préférence de 28 à 36 inclus;

lorsque X ou Y désigne sulfoxyde, Y ou X ne désigne pas soufre.

Les composés utilisés préférentiellement conformément à l'invention, sont ceux dans lesquels X désigne oxygène, Y désigne méthylène, et $R_1$ et $R_2$ désignent des radicaux ayant 12 à 18 atomes de carbone.

Les composés de formule (I) sont obtenus par réaction d'un composé à hydrogène actif de formule (II) :

$$R_1XH$$

avec un composé comportant un groupement oxirane terminal et répondant à la formule (III) :

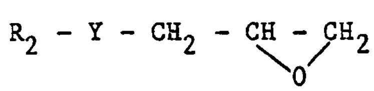

(III)

Lorsque X désigne oxygène, ces réactions sont réalisées de préférence en présence d'un excès molaire du composé de formule (II) par rapport au composé de formule (III), cet excès pouvant atteindre cinq fois la

quantité stoechiométrique, en présence d'un catalyseur acide tel que par exemple le trifluorure de bore, $SnCl_4$ ou $ZnCl_2$ ou en présence d'un catalyseur alcalin tel que le sodium, le potassium, le méthylate, l'éthylate ou le tertiobutylate de sodium ou de potassium.

En catalyse acide, la température de réaction est comprise entre 40°C et 100°C; elle est comprise de préférence, en catalyse alcaline entre 80° et 180°C.

Lorsque l'un des groupements X ou Y désigne un atome de soufre, la réaction est réalisée de préférence en catalyse alcaline et avec des proportions stoechiométriques des composés de formules (II) et (III).

La proportion de catalyseur utilisée est généralement de 0,1 à 3% en poids par rapport au poids de la masse réactionnelle.

Selon le sens d'ouverture de l'époxyde de formule (III), on peut obtenir les isomères de formules (Ia) et (Ib) suivantes :

$$R_1 - X - CH_2 - \underset{\displaystyle OH}{\underset{|}{CH}} - CH_2 - Y - R_2 \qquad (Ia)$$

$$R_1 - X - \underset{\displaystyle CH_2OH}{\underset{|}{CH}} - CH_2 - Y - R_2 \qquad (Ib)$$

Les composés de formule (I) peuvent être purifiés, après élimination sous pression réduite du composé à hydrogène actif de formule (II) résiduel, par distillation moléculaire sous environ $10^{-3}$mm de mercure (0,13 Pascal).

Lorsque le composé de départ de formule (II) est un alcool, il est également possible d'utiliser, dans le cadre de l'invention, le produit de réaction après simplement élimination partielle ou totale de l'excès d'alcool de formule (II), voire le produit brut de réaction, c'est-à-dire en conservant l'excès d'alcool gras de formule (II) dans sa totalité. En effet, les alcools gras de formule (II) utilisés dans le cadre de la préparation des composés de formule (I) utilisés conformément à l'invention ne sont pas préjudiciables aux propriétés attendues des composés de formule (I) et ils peuvent contribuer à la stabilisation et à l'opacification des compositions détergentes.

Les composés de formule (I) dans laquelle au moins un des groupements X ou Y désigne un atome de soufre peuvent être oxydés, selon des procédés classiques, en sulfoxydes qui répondent à la formule (IV) :

$$R_1 - X_1 \{ C_2H_3(OH) \} CH_2 - Y_1 - R_2 \qquad (IV)$$

dans laquelle $R_1$ et $R_2$ ont la même signification que dans la formule (I); $X_1$ désigne un atome d'oxygène ou un groupement sulfoxyde; $Y_1$ désigne un atome d'oxygène, un groupement méthylène ou sulfoxyde, au moins un des symboles $X_1$ ou $Y_1$ représente un groupement sulfoxyde.

Les silicones utilisées conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, elles sont choisies parmi celles possédant un point d'ébullition compris entre 60°C et 260°C et plus particulièrement parmi :

(i) les silicones cycliques comportant de 3 à 7 atomes de silicium et de préférence 4 à 5. Il s'agit, par exemple, de l'octaméthylcyclotétrasiloxane vendu sous le nom de "VOLATILE SILICONE 7207" par UNION CARBIDE ou "SILBIONE 70045 V 2" par RHONE POULENC, le décaméthylcyclopentasiloxane vendu sous le nom de "VOLATILE SILICONE 7158" par UNION CARBIDE, "SILBIONE 70045 V 5" par RHONE POULENC, ainsi que leurs mélanges.

On cite également les cyclocopolymères du type diméthylsiloxane/méthylalkylsiloxane, tel que la "SILICONE VOLATILE FZ 3109" vendue par la Société UNION CARBIDE, de structure chimique :

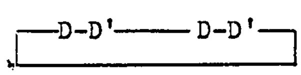

$$\text{avec D : } -\underset{\displaystyle CH_3}{\overset{\displaystyle CH_3}{\overset{|}{\underset{|}{Si}}}}-O- \qquad D' : -\underset{\displaystyle C_8H_{17}}{\overset{\displaystyle CH_3}{\overset{|}{\underset{|}{Si}}}}-O-$$

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcylotétrasiloxane et d'oxy-1,1'(hexa-2,2,2',2',3,3'-triméthylsilyloxy)bisnéopentane;

(ii) les silicones volatiles linéaires ayant 2 à 9 atomes de silicium et possédant une viscosité inférieure ou égale à $5.10^{-6}m^2/s$ à 25°C. Il s'agit, par exemple, du décaméthyltétrasiloxane vendu sous la dénomination "SH 200" par la Société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionels ainsi que leurs mélanges.

Ces silicones sont plus particulièrement choisis parmi les polyalkylsiloxanes parmi lesquels on peut citer principalement les polydiméthylsiloxanes linéaires à groupements terminaux triméthylsilyle ayant une viscosité de $5.10^{-6}$ à 2,5 $m^2/s$ à 25°C et de préférence $1.10^{-5}$ à 1 $m^2/s$.

Parmi ces polyalkylsiloxanes, on peut citer à titre non limitatif les produits commerciaux suivants :

- les huiles SILBIONE des séries 47 et 70 047 commercialisées par RHONE POULENC telles que par exemple l'huile 47 V 500 000;
- les huiles de la série 200 de la Société DOW CORNING;
- les huiles VISCASIL de GENERAL ELECTRIC et certaines huiles des séries SF (SF 96, SF 18) de GENERAL ELECTRIC.

On peut également citer les polydiméthylsiloxanes linéaires à groupements terminaux diméthylsilanol tels que les huiles de la série 48 de la Société RHONE POULENC.

Dans cette classe de polyalkylsiloxanes, on peut également citer les produits vendus sous les dénominations "ABIL WAX 9800 et 9801" par la Société GOLDSCHMIDT qui sont des polyalkyl($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2}$ $m^2/s$ à 25°C.

Parmi ces polyalkylarylsiloxanes on peut citer à titre d'exemple les produits commercialisés sous les dénominations suivantes :

. les huiles SILBIONE de la série 70 641 de RHONE POULENC;
. les huiles des séries RHODORSIL 70 633 et 763 de RHONE POULENC;
. l'huile DC 556 COSMETIC GRAD FLUID de DOW CORNING;
. les silicones de la série PK de BAYER comme le produit PK20;
. les silicones des séries PN, PH de BAYER comme les produits PN 1000 et PH 1000;
. certaines huiles des séries SF de GENERAL ELCTRIC telles que SF 1023, SF 1154, SF 1250, SF 1265.

Les gommes de silicone utilisables conformément à l'invention sont notamment des polydiorganosiloxanes ayant des masses moléculaires élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On peut plus particulièrement citer les produits suivants :

- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 vendu par la Société DOW CORNING;
. les mélanges formés à partir d'une gomme polydiméthylsiloxane avec une silicone cyclique tel que le produit SF 1214 Silicone Fluid de la Société GENERAL ELECTRIC, ce produit est une gomme SE 30 correspondant à une diméthicone, ayant un poids moléculaire de 500 000 solubilisée dans l'huile SF 1202 Silicone Fluid correspondant au décaméthylcyclopentasiloxane;
. les mélanges de deux PDMS de viscosités différentes, et plus particulièrement d'une gomme PDMS et d'une huile PDMS, tels que le produit SF 1236 de la Société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}m^2/s$. Ce produit comporte de préférence 15% de gomme SE 30 et 85% d'une

huile SF 96.

Les résines d'organopolysiloxanes utilisables conformément à l'invention sont des systèmes siloxaniques réticulés renfermant les unités :

$R_2SiO_{2/2}$, $RSiO_{3/2}$ et $SiO_{4/2}$ dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

On peut citer parmi ces résines le produit vendu sous la dénomination "DOW CORNING 593" ou ceux vendus sous les dénominations "SILICONE FLUID SS 4230 et SS 4267" par la Société GENERAL ELECTRIC et qui sont des siloxanes de structure diméthyl/triméthyl siloxane.

Les silicones organo modifiées utilisables conformément à l'invention sont des silicones telles que définies précédemment et comportant dans leur structure un ou plusieurs groupements organofonctionnels directement fixés sur la chaîne siloxanique ou fixés par l'intermédiaire d'un radical hydrocarboné.

Parmi ces silicones on peut citer les silicones comportant :

. des groupements polyéthylèneoxy et/ou polypropylèneoxy comportant éventuellement des groupements alkyle tels que le produit dénommé diméthicone copolyol vendu par la Société DOW CORNING sous la dénomination DC 1248 et l'alkyl ($C_{12}$) méthicone copolyol vendue par la Société DOW CORNING sous la dénomination Q2 5200; les huiles SILWET L 722, L 7500, L 77, L 711 de la Société UNION CARBIDE;

. des groupements aminés substitués ou non comme les produits vendus sous la dénomination GP4 Silicone Fluid et GP 7100 par la Société GENESEE ou les produits vendus sous les dénominations Q2 8220 et DC 929 par la Société DOW CORNING. Les groupements aminés substitués sont en particulier des groupements aminoalkyle en $C_1$-$C_4$;

. des groupements thiols comme les produits vendus sous les dénominations "GP 72 A" et "GP 71" de GENESEE;

. des groupements carboxylates comme dans les produits décrits dans le brevet EP 186 507 de la Société CHISSO CORPORATION;

. des groupements alcoxylés comme le produit vendu sous la dénomination "SILICONE COPOLYMER F-755" par SWS SILICONES et ABIL WAX 2428, 2434 et 2440 par la Société GOLDSCHMIDT;

. des groupements hydroxylés comme les polyorganosiloxanes à fonction hydroxyalkyle décrits dans la demande de brevet français FR-A-85 163 34 répondant à la formule (V) :

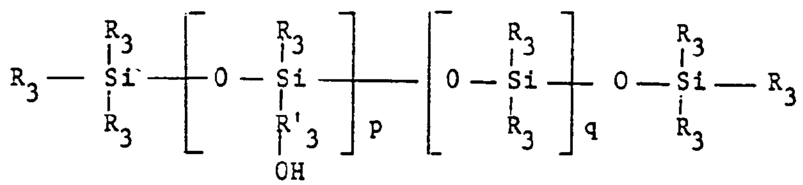

(V)

dans laquelle les radicaux $R_3$ identiques ou différents sont choisis parmi les radicaux méthyle et phényle; au moins 60% en mole des radicaux $R_3$ désignant méthyle; le radical $R'_3$ est un chaînon alkylène divalent hydrocarboné en $C_2$-$C_{18}$; p est compris 1 et 30 inclus; q est compris entre 1 et 150 inclus;

. des groupements acyloxyalkyle tels que par exemple les polyorganosiloxanes décrits dans la demande de brevet français FR-A 88 17 433 et répondant à la formule (VI)

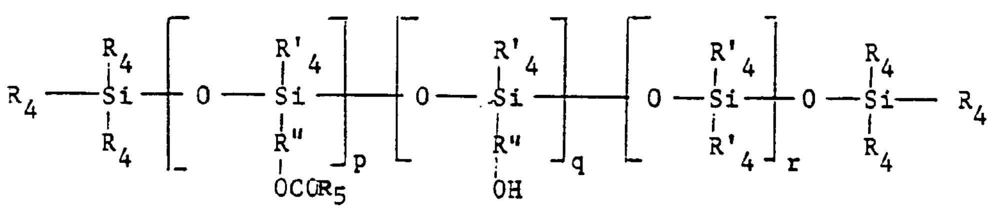

(VI)

dans laquelle :

$R_4$ désigne un groupement méthyle, phényle, $-OCOR_5$, hydroxyle, un seul des radicaux $R_4$ par atome de silicium pouvant être OH;

$R'_4$ désigne méthyle, phényle; au moins 60% en proportion molaire de l'ensemble des radicaux $R_4$ et

R'$_4$ désignant méthyle;

$R_5$ désigne alkyle ou alcényle en $C_8$-$C_{20}$;

R" désigne un radical alkylène hydrocarboné divalent, linéaire ou ramifié en $C_2$-$C_{18}$;

r est compris entre 1 et 120 inclus;

p est compris entre 1 et 30;

q est égal à 0 ou est inférieur à 0,5 p, p+q étant compris entre 1 et 30; les polyorganosiloxanes de formule (VI) peuvent contenir des groupements

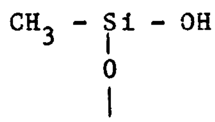

dans des proportions ne dépassant pas 15% de la somme p+q+r.

Les composés de formule (VI) peuvent être préparés par estérification de polyorganosiloxanes à fonction hydroxyalkyle de formule (V) ci-dessus.

L'estérification s'effectue de façon connue avec un acide $R_5$ COOH ou l'anhydride d'acide à une température comprise entre 100 et 250°C en présence éventuellement d'un catalyseur comme le chlorure d'aluminium ou le chlorure de zinc ou d'un acide fort comme l'acide chlorhydrique ou l'acide sulfurique.

On peut également effectuer une transestérification par chauffage à 100-150°C d'un ester méthylique de formule $R_5COOCH_3$ et d'un diorganopolysiloxane de formule (V), en présence d'un catalyseur acide comme l'acide paratoluène sulfonique ou une terre acide de type Montmorillonite telle que par exemple le produit vendu sous la dénomination "KATALYSATOR KSF/0" par la Société SUD-CHEMIE;

- des groupements anioniques de type carboxylique tels que les groupements alkylcarboxyliques comme ceux présents dans le produit X-22-3701E de la société SHIN-ETSU; 2-hydroxyalkylsulfonate; 2-hydroxyalkylthiosulfate tels que les produits vendus par la Société GOLDSCHMIDT sous les dénominations "ABIL S201" et "ABIL S 255".
- des groupements hydroxyacylamino, comme les polyorganosiloxanes décrits dans la demande EP 342 834. on peut citer par exemple le produit Q2-8413 de la Société DOW CORNING

Les polyorganosiloxanes particulièrement préférés conformément à l'invention sont :

- les silicones non volatiles choisies dans la famille des polyalkylsiloxanes linéaires à groupements terminaux triméthylsilylés telles que les huiles ayant une viscosité comprise entre 0,2 et 2,5 $m^2/s$ à 25°C des séries SILBIONE 70047 et 47 et plus particulièrement l'huile 47 V 500 000 commercialisées par la Société RHONE POULENC, ou les polyalkylarylsiloxanes tels que l'huile SILBIONE 70641 V 200 commercialisée par la Société RHONE POULENC;
- des mélanges d'organosiloxanes et de silicones cycliques tels que le produit Q2 1401 vendu par la Société DOW CORNING, et le produit SF 1214 vendu par la Société GENERAL ELECTRIC;

Les mélanges de deux PDMS de viscosités différentes notamment d'une gomme et d'une huile tels que le produit SF 1236 vendu par la Société GENERAL ELECTRIC

- la résine d'organopolysiloxane vendue sous la dénomination DOW CORNING 593.

Les agents tensio-actifs utilisés dans les compositions de lavage conformes à l'invention sont choisis parmi les agents tensio-actifs anioniques, amphotères, zwittérioniques, non ioniques ou leurs mélanges ayant des propriétés détergentes.

Parmi ces agents tensio-actifs anioniques on peut citer plus particulièrement : les sels alcalins, les sels d'ammonium, les sels d'amines, les sels d'aminoalcools, les sels de magnésium des composés suivants :

- les alkylsulfates, alkyléther sulfates, alkylamidoéther-sulfates, alkylarylpolyéthersulfates, monoglycérides sulfates;
- les alkylsulfonates, alkylamides sulfonates, alkylarylsulfonates, oléfines sulfonates, paraffines sulfonates;
- les alkylsulfosuccinates, les alkyléthersulfosuccinates, les alkylamides sulfosuccinates;
- les alkylsulfosuccinamates;
- les alkylsulfoacétates;
- les alkylphosphates, alkyléther phosphates;
- les acylsarcosinates, les acyliséthionates, N-acyltaurates.

Le radical alkyle ou acyle de ces différents composés est généralement constitué par une chaîne carbonée comportant de 12 à 20 atomes de carbone.

Parmi les agents tensio-actifs anioniques, on peut également citer : les sels d'acide gras tels que des acides oléique, ricinoléïque, palmitique, stéarique; les acides d'huile de coprah ou d'huile de coprah hydrogénée; les acyl lactylates dont le radical acyle comporte 8 à 20 atomes de carbone.

D'autres agents tensio-actifs anioniques sont constitués par des acides éthers carboxyliques polyoxyalkylénés de formule :

$$R_6\text{-}(OC_3H_6)_p\text{-}(OC_2H_4)_n\text{-}OCH_2COOA \qquad (VII)$$

dans laquelle :

$R_6$ désigne un radical ou un mélange de radicaux alkyle ou alcényle en $C_8$-$C_{22}$, linéaire ou ramifié, alkyl($C_8$-$C_9$)phényle, $R_7$CONH-$CH_2$-$CH_2$- avec $R_7$ désignant un radical alkyle ou alcényle, linéaire ou ramifié, en $C_{11}$-$C_{21}$;

n est un nombre entier ou décimal compris entre 2 et 24,

p est un nombre entier ou décimal compris entre 0 et 6,

A désigne un atome d'hydrogène ou bien Na, K, Li, 1/2 Mg ou un reste monoéthanolamine, ammonium ou triéthanolamine.

Les acides éthers carboxyliques polyoxyalkylénés utilisables conformément à l'invention, sont choisis de préférence parmi les composés de formule (VII) dans laquelle $R_6$ désigne un radical ou un mélange de radicaux alkyle ($C_{10}$-$C_{18}$), oléyle; un radical nonylphényle ou octylphényle, A désigne un atome d'hydrogène ou de sodium et p=0.

On utilise de préférence les produits commerciaux vendus par la Société CHEM Y sous les dénominations AKYPO ou par la Société SANDOZ sous les dénominations "SANDOPAN".

Parmi les agents tensio-actifs non ioniques on peut citer plus particulièrement : les alcools, les alkylphénols et les acides gras polyéthoxylés, polyoxypropylénés ou polyglycérolés à chaîne grasse comportant 8 à 18 atomes de carbone. Le nombre de groupements oxyde d'éthylène, oxyde de propylène étant compris entre 2 et 50 et le nombre de groupements glycérol étant compris entre 2 et 30.

On peut également citer les copolymères d'oxydes d'éthylène et de propylène, les condensats d'oxydes d'éthylène et de propylène sur des alcools gras, les amides gras polyéthoxylés (de préference à 2 à 30 moles d'oxyde d'éthylène), des amines grasses polyéthoxylées (de préférence de 2 à 30 moles d'oxyde d'éthylène), des éthanolamides, des esters d'acides gras du sorbitan oxyéthylénés (de préférence 2 à 30 moles d'oxyde d'éthylène) ou non, des esters d'acides gras du polyéthylène glycol, des triesters phosphoriques, des esters d'acides gras de sucrose, les alkylpolyglycosides, des oxydes d'amines grasses tels que les oxydes d'alkylamines ou de N-acylamidopropylmorpholine.

Les alcools gras oxyéthylénés ou polyglycérolés préférés sont l'alcool oléique oxyéthyléné à 10 moles d'oxyde d'éthylène, l'alcool laurique oxyéthyléné à 12 moles d'oxyde d'éthylène, le nonylphénol oxyéthyléné à 9 moles d'oxyde d'éthylène, l'alcool oléique polyglycérolé à 4 moles de glycérol.

D'autres composés entrant dans cette classe sont des composés répondant aux formules (VIII) à (X) ci-après et/ou parmi les composés préparés selon les procédés décrits ci-après.

a) les composés de formule :

$$R_8O\text{-}(CH_2\text{-}\underset{\displaystyle CH_2OH}{\underset{|}{CH}}\text{-}O)_m\text{-}H \qquad (VIII)$$

dans laquelle $R_8$ désigne un radical ou un mélange de radicaux alkyle ayant 10 à 14 atomes de carbone et m est un nombre statistique entier ou décimal de 2 à 10 et de préférence de 3 à 6. Ces composés peuvent être préparés selon le procédé décrit dans le brevet FR-A-1 477 048;

b) les dérives de formule :

$$R_9\text{-}CONH\text{-}CH_2\text{-}CH_2\text{-}O\text{-}CH_2\text{-}CH_2\text{-}O(CH_2\text{-}CHOH\text{-}CH_2\text{-}O)_n\text{-}H \qquad (IX)$$

dans laquelle $R_9$ désigne un radical ou un mélange de radicaux alkyle et/ou alcényle de 11 à 17 atomes de carbone et n désigne un nombre statistique entier ou décimal de 1 à 5 et de préférence 1,5 à 4. Ces composés de formule (IX) peuvent être préparés selon le procédé décrit dans le brevet français FR-A-2 328 763;

c) les composés de formule :

$$R_{10}\text{-}CHOH\text{-}CH_2\text{-}O(CH_2\text{-}CHOH\text{-}CH_2\text{-}O)_p\text{-}H \qquad (X)$$

dans laquelle $R_{10}$ désigne un radical aliphatique, cycloaliphatique, arylaliphatique ayant de préférence 7 à 21 atomes de carbone et leurs mélanges; les chaînes aliphatiques désignant en particulier des chaînes alkyle peuvent comporter de 1 à 6 groupements éther, thioéther et/ou hydroxyméthylène et p a une valeur

statistique de 2 à 10 inclus.

Ces composés sont préparés par condensation en catalyse alcaline de 2 à 10 moles, et de préférence de 2,5 à 6 moles de glycidol, sur un alpha-diol ou un mélange d'alpha-diols en $C_9$-$C_{23}$ à la température de 120-180°C et de préférence de 140 à 160°C, le glycidol étant ajouté lentement selon le procédé de préparation décrit dans le brevet FR-A-2 091 516;

d) les composés préparés par condensation en catalyse acide de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou l'alpha-diol. Le procédé de préparation de ces composés est décrit dans le brevet FR-A-2 169 787;

e) les composés de poly(hydroxypropyléther) préparés par polyaddition de monochlorhydrine du glycérol sur un composé organique à chaîne grasse poly(hydroxylé) en présence d'une base forte, par élimination au fur et à mesure de l'eau par distillation, décrits plus particulièrement dans le brevet FR-A-2 574 786.

Parmi les agents tensio-actifs non ioniques de la famille des poly(hydroxypropyléther) décrits dans les paragraphes (a), (b), (c), (d), et (e) ci-dessus les composés préférés sont représentés par les formules :

$$C_{12}H_{25}O\text{-}(CH_2\text{-}\underset{\displaystyle CH_2OH}{\underset{|}{CH}}\text{-}O)_{4,2}H \qquad (XI)$$

$$R_8O\text{-}(CH_2\text{-}\underset{\displaystyle CH_2OH}{\underset{|}{CH}}\text{-}O)_{3,75}H \qquad (XII)$$

où $R_8$ désigne un mélange de radicaux alkyle $C_{10}H_{21}$ et $C_{12}H_{25}$;

- les composés préparés par condensation en catalyse alcaline de 3,5 moles de glycidol sur un alpha-diol ayant 12 atomes de carbone selon le procédé décrit dans le brevet FR-A-2 091 516; et les composés répondant à la formule :

  $$R_{10}\text{-CONH-}CH_2\text{-}CH_2\text{-O-}CH_2\text{-}CH_2\text{-O-}(CH_2\text{-CHOH-}CH_2\text{-O})_{3,5}H \qquad (XIII)$$

  où $R_{10}$ désigne un mélange de radicaux comprenant des radicaux alkyles et alcényles suivants : $C_{11}H_{23}$, $C_{13}H_{27}$ et les radicaux dérivés des acides gras du coprah, des radicaux dérivés de l'acide oléïque;
- les composés préparés par condensation de 3,5 moles de glycidol sur un mélange d'alpha-diols en $C_{11}$-$C_{14}$ tel que décrit dans le brevet FR-A-2 091 516, et l'agent tensio-actif non ionique de poly(hydroxypropyléther) obtenu par condensation de la monochlorhydrine du glycérol (2,5 moles) en présence de soude sur le dodécanediol-1,2 sont particulièrement préférés.

Parmi les agents tensio-actifs amphotères et zwittérioniques pouvant être utilisés, on peut citer à titre d'exemple :

1. les dérivés d'amines secondaires ou tertiaires aliphatiques dans lesquels le radical aliphatique est une chaîne linéaire ou ramifiée comportant de 8 à 18 atomes de carbone et qui contient au moins un groupe anionique hydrosolubilisant carboxylique, sulfonate, sulfate, phosphate ou phosphonate;
2. les alkylbétaïnes, les sulfobétaïnes, les amidobétaïnes ou les amidosulfobétaïnes.

Parmi ces composés, on peut citer plus particulièrement les produits vendus sous la dénomination "MIRANOL" décrits en particulier dans les brevets US-A-2 528 378 et 2 781 354 et classés dans le dictionnaire CTFA (3ème édition 1982) sous la dénomination d'"AMPHOCARBOXYGLYCINATES et d'AMPHOCARBOXYPROPIONATES".

Ces produits présentent les structures suivantes : pour les AMPHOCARBOXYGLYCINATES :

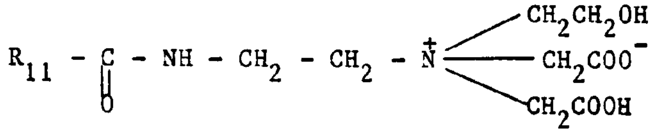

(XIV)

dans laquelle $R_{11}$ désigne un radical alkyle dérive du coprah, un radical heptyle, nonyle ou undécyle;

pour les AMPHOCARBOXYPROPIONATES :

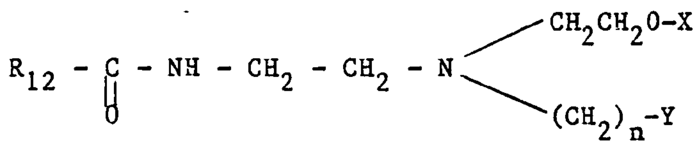

(XV)

n = 1 ou 2;
X désigne $-CH_2CH_2COOH$ ou hydrogène;
Y désigne -COOH ou le radical

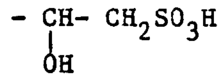

$R_{12}$ désigne un radical alkyle dérivé du coprah, un radical alkyle en $C_7$, $C_9$, $C_{11}$, $C_{13}$ un radical alkyle en $C_{17}$ et sa forme iso, un radical en $C_{17}$ insaturé, un radical alkyle dérivé de l'huile de lin;

Les alkylbétaïnes sont choisies de préférence parmi les alkyl-($C_{10}$-$C_{20}$) bétaïnes.

Dans les compositions conformes à l'invention, on utilise de préférence des mélanges d'agents tensio-actifs et en particulier des mélanges d'agents tensio-actifs anioniques et d'agents tensio-actifs amphotères, zwitterioniques ou non ioniques. Un mélange particulièrement préféré est un mélange constitué d'un agent tensioactif anionique et d'un agent tensio-actif zwittérionique.

On utilise de préférence un agent tensio-actif anionique choisi parmi les alkyl($C_{12}$-$C_{14}$)sulfates de sodium, de triéthanolamine ou d'ammonium, les alkyl($C_{12}$-$C_{14}$)éthersulfates de sodium oxyéthylénés à 2,2 moles d'oxyde d'éthylène, le cocoyl iséthionate de sodium et l' α-oléfine($C_{14}$-$C_{16}$) sulfonate de sodium et leurs mélanges avec

- soit un agent tensio-actif amphotère tel que l'amphocarboxyglycinate défini par la formule (XIV) ci-dessus dans laquelle $R_{11}$ désigne un radical alkyle dérivé du coprah, dénommé cocoamphocarboxyglycinate vendu par la Société MIRANOL sous la dénomination commerciale "MIRANOL C2M CONC en solution aqueuse à 38% de matière active;
- soit un agent tensio-actif zwittérionique tel que la laurylbétaïne vendue sous la dénomination "DEHYTON AB 30" en solution aqueuse à 32% de MA par la Société HENKEL.

Les silicones sont utilisées dans les compositions conformes à l'invention dans des proportions comprises de préférence entre 0,05 et 20% et de préférence entre 0,1 et 10% en poids par rapport au poids total de la composition.

Les alcools à groupement(s) éther et/ou thioéther ou sulfoxyde répondant à la formule (I) ou à la formule (IV) utilisés conformément à l'invention sont présents dans des proportions suffisantes pour assurer l'homogénéité, l'épaississement et l'opalescence des compositions et de préférence dans des proportions comprises entre 0,1 et 10% par rapport au poids total de la composition et en particulier entre 0,5 et 5%.

Les agents tensio-actifs sont utilisés dans les compositions conformes à l'invention dans des proportions suffisantes pour conférer un caractère détergent à la composition et sont comprises de préférence entre 5 et 50% par rapport au poids total de la composition et en particulier entre 8 et 35%.

Le pH de ces compositions est généralement compris entre 3 et 9 et plus particulièrement entre 4 et 8.

Le milieu aqueux peut être constitué uniquement par de l'eau ou par un mélange d'eau et d'un solvant cosmétiquement acceptable tel qu'un alcool inférieur en $C_1$-$C_4$, comme l'éthanol, l'isopropanol, le n-butanol; les alkylèneglycols comme l'éthylèneglycol, les éthers de glycols.

Les compositions conformes à l'invention, peuvent contenir en plus de l'association définie ci-dessus des agents régulateurs de viscosité tels que des électrolytes, des hydrotropes ou d'autres agents épaississants. On peut citer en particulier le chlorure de sodium, le xylène sulfonate de sodium, les scléroglucanes, les gommes de xanthane, les alcanolamides d'acide de coprah, les alcanolamides d'acide alkyl éther carboxylique éventuellement oxyéthylénés avec jusqu'à 5 moles d'oxyde d'éthylène tel que le produit vendu sous la dénomination "AMINOL A15" par la Société CHEM Y. Ces agents régulateurs de viscosité sont utilisés dans les compositions selon l'invention dans des proportions pouvant aller jusqu'à 10% en poids par rapport au poids total de la composition.

Les compositions conformes à l'invention peuvent également contenir jusqu'à 3% d'agents nacrants ou opacifiants bien connus dans l'état de la technique tels que par exemple les palmitates de sodium ou de magnésium, les stéarates et hydroxystéarates de sodium ou de magnésium, les monostéarate ou distéarate d'éthylène glycol.

Les compositions conformes à l'invention peuvent éventuellement contenir en outre d'autres agents ayant pour effet d'améliorer les propriétés cosmétiques des cheveux ou de la peau sans cependant altérer la stabilité des compositions. On peut citer à ce sujet les agents tensio-actifs cationiques, des polymères anioniques ou non ioniques ou cationiques ou amphotères ou des protéines éventuellement quaternisées.

Les polymères cationiques sont choisis parmi les polymères comportant des groupements amines primaires, secondaires, tertiaires et/ou quaternaires faisant partie de la chaîne polymère ou directement reliés à celle-ci, ayant un poids moléculaire compris entre 500 et environ 5 000 000.

On peut citer à titre non limitatif :

- les polymères de polyammonium quaternaires tels que ceux décrits dans la demande EP-A-122 324 ou dans les brevets français 2 333 012, 2 270 846, 2 270 851, 2 471 777, 2 316 271, 2 331 323, ou le brevet US 4 157 388.

Les polyammoniums quaternaires particulièrement préférés sont ceux comportant les motifs :

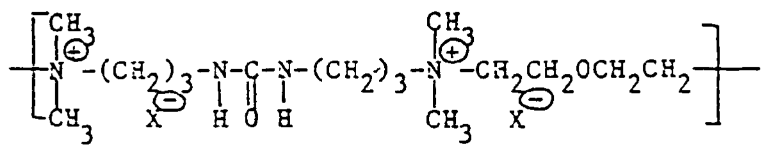

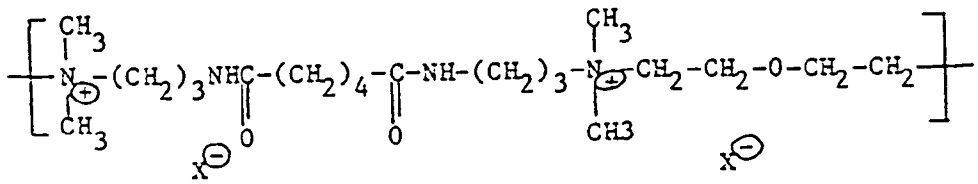

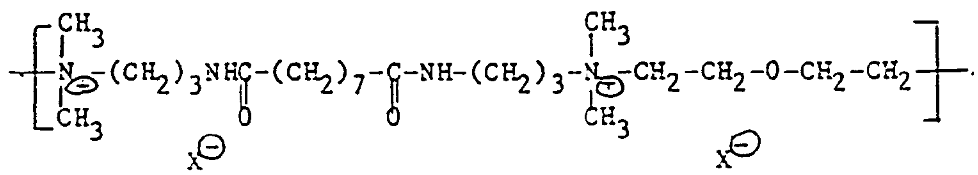

Ces polyammoniums quaternaires sont vendus sous la dénomination MIRAPOL A15, MIRAPOL AD1, MIRAPOL AZ1 ou MIRAPOL 175 par la Société MIRANOL.

On peut également citer comme polymères cationiques, les polysaccharides cationiques tels que ceux décrits dans les brevets US 3 589 578 et 4 031 307 comme le produit commercialisé sous la dénomination JAGUAR C 13S par MEYHALL.

A titre de polymère amphotère on peut citer :

- les polymères comportant de préférence environ 60 à environ 99% de motifs dérivés d'un monomère de diallyl dialkylammonium, dans lequel les groupements alkyle comportent de 1 à 18 atomes de carbone et de préférence environ 1 à environ 40% de motifs dérivés de monomères choisis parmi les acides acrylique et méthacrylique. Le poids moléculaire moyen de ces polymères, déterminé par chromatographie par perméation de gel, est compris entre 50 000 et 1000 000.

  Les polymères préférés sont les polymères de diméthyldiallyl- ou de diéthyldiallylammonium et d'acide acrylique tels que le produit commercialisé sous la dénomination MERQUAT 280 par la société MERCK;
- les polymères dérivés du chitosane tels que ceux décrits dans le FR 2 137 684 et comportant des motifs répondant aux formules :

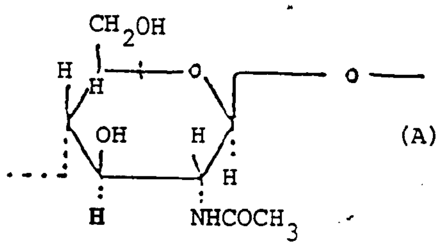

(A)

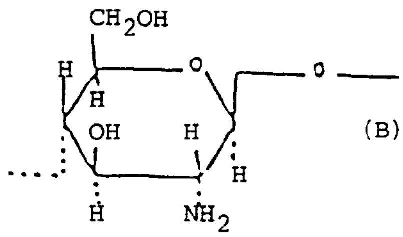

(B)

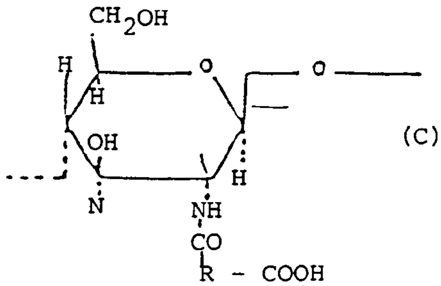

(C)

dans lesquelles le motif A est présent dans des proportions de 0 à 30% en poids, le motif B de 5 à 50% en poids et le motif C dans des proportions de 30 90% en poids; R représente un groupement alkylène linéaire ou ramifié comportant de 2 à 5 atomes de carbone.

Les polymères préférés comportent de 0 à 20% de motifs A 40 à 50% de motifs B et 40 à 50% de motifs C et R désigne $-CH_2-CH_2-$

Elles peuvent contenir également des synergistes de mousses tels que des 1,2-alcanediols en $C_{10}$-$C_{18}$ ou des alcanolamides gras dérivés de mono ou de diéthanolamine.

Ces compositions peuvent également contenir différents adjuvants utilisés couramment en cosmétique tels que des parfums, des conservateurs, des agents séquestrants, des stabilisateurs de mousse et des agents acidifiants ou alcalinisants bien connus en cosmétique.

Les compositions selon l'invention sont utilisés de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage des cheveux et de la peau, auquel cas ils sont appliqués sur la peau et les cheveux humides et sont rincés après application.

Les exemples suivants sont destinés à illustrer l'invention.

EXEMPLE 1

On prépare un shampooing d'aspect opaque de composition suivante :

| | |
|---|---|
| - Akyl($C_{12}$-$C_{14}$)éther sulfate de sodium oxyéthyléné à 2,2 moles d'oxyde d'éthylène vendu à 28% de MA | 14 g MA |
| - Laurylbétaïne vendue par la société HENKEL sous la dénomination commerciale "DEHYTON AB 30" en solution aqueuse à 32% de MA | 2,6 g MA |
| - Cocoylisethionate de sodium vendu sous la dénomination "ARLATONE SCI Prilled" par la société ICI | 6 g |
| - Alcool linéaire ($C_{12}$-$C_{14}$) oxyéthyléné à 3 moles d'oxyde d'éthylène | 10 g |
| - Huile 47 V 500000 vendue par la société RHONE POULENC | 3 g |
| - Composé de formule (I) dans laquelle $R_1 = C_{12}H_{25}$ X = O, Y = $CH_2$ $R_2 = C_{12}H_{25}/C_{14}H_{29}$ (50/50 en mole) préparé par réaction de 3 moles d'alcool sur une mole d'époxyde puis distillation de l'alcool excédentaire | 3 g |
| - Conservateur, parfum qs | |
| - pH spontané 5,7 | |
| - Eau qsp | 100 g |

Les cheveux lavés avec cette composition de shampooing moussante et bien détergente se peignent facilement, sont brillants et présentent un toucher doux.

EXEMPLE 2

On prépare un shampooing d'aspect opaque de composition suivante :

| | | |
|---|---|---|
| - Akyl($C_{12}$-$C_{14}$)éther sulfate de sodium oxyéthyléné à 2,2 moles d'oxyde d'éthylène vendu à 28% de MA | 14 | g MA |
| - Laurylbétaïne vendue par la société HENKEL sous la dénomination commerciale "DEHYTON AB 30" en solution aqueuse à 32% de MA | 2,6 | g MA |
| - Cocoylisethionate de sodium vendu sous la dénomination "ARLATONE SCI Prilled" par la société ICI | 6 | g |
| - Huile 47 V 500000 vendue par la société RHONE POULENC | 3 | g |
| - Composé de formule (I) dans laquelle $R_1 = C_{14}H_{29}$, $R_2 = C_{12}H_{25}$ X = O, Y = $CH_2$ préparé par réaction de 3 moles d'alcool sur une mole d'époxyde suivie d'une distillation de l'alcool excédentaire et d'une distillation du composé de formule (I) | 2,5 | g |
| - Monoéthanolamide d'acide alkyl($C_{13}$-$C_{15}$)éther-carboxylique oxyéthyléné à 2 moles d'oxyde d'éthylène vendu sous la dénomination "AMINOL A 15" par la société CHEM Y | 2 | g |
| - Conservateur, parfum qs | | |
| - pH spontané 5,2 | | |
| - Eau qsp | 100 | g |

Les cheveux lavés avec cette composition de shampooing moussante et bien détergente se peignent facilement, sont brillants et présentent un toucher doux.

EXEMPLE 3

On prépare un shampooing d'aspect opaque de composition suivante :

| | |
|---|---|
| - Akyl($C_{12}$-$C_{14}$)éther sulfate de sodium oxyéthyléné à 2,2 moles d'oxyde d'éthylène vendu à 28% de MA | 14 g MA |
| - Monosulfosuccinate d'alcool laurique, sel disodique vendu en solution aqueuse à 40% de MA par la société ZSCHIMMER et SCHWARZ sous la dénomination "SETACIN F Spécial Paste" | 2,4 g MA |
| - Laurylbétaïne vendue par la société HENKEL sous la dénomination commerciale "DEHYTON AB 30" en solution aqueuse à 32% de MA | 2,6 g MA |
| - Huile 47 V 500000 vendue par la société RHONE POULENC | 3 g |
| - Composé de formule (I) dans laquelle $R_1 = C_{16}H_{33}$, $R_2 = C_{14}H_{29}$ X = O, Y = $CH_2$ préparé par réaction de 3 moles d'alcool sur une mole d'époxyde, utilisé brut | 2,5 g |
| - Monoéthanolamide d'acide alkyl($C_{13}$-$C_{15}$)éther-carboxylique oxyéthyléné à 2 moles d'oxyde d'éthylène vendu sous la dénomination "AMINOL A 15" par la société CHEM Y | 3 g |
| - Chlorure de sodium | 0,2 g |
| - Conservateur, parfum qs | |
| - pH spontané 5,4 | |
| - Eau qsp | 100 g |

Les cheveux lavés avec cette composition de shampooing moussante et bien détergente se peignent facilement, sont brillants et présentent un toucher doux.

EXEMPLE 4

On prépare un shampooing de composition suivante :

| | | | |
|---|---|---|---|
| - Polysiloxane à fonction hydroxyacylamino vendu sous la dénomination Q2-8413 par la société DOW CORNING | | | 4 g |
| - Hydroxyéthylcellulose quaternisée vendue sous la dénomination JR 400 par la société UNION CARBIDE | | | 0,1 g |
| - Lauryléthersulfate de sodium oxyéthyléné à 2 moles d'oxyde d'éthylène à 28% de MA | | | 15 g MA |
| - Cocoylbétaïne à 32% de MA | | | 2,4 g MA |
| - Distéarate d'éthylèneglycol | | | 2 g |
| - Composé de formule $C_{16}H_{33}-O-C_2H_3(OH)-CH_2-C_{14}H_{29}$ préparé par réaction de 3 moles d'alcool sur une mole d'époxyde , utilisé brut | | | 2,5 g |
| - Triéthanolamine | qs | pH 7,4 | |
| - Eau | qsp | | 100 g |

EXEMPLE 5

On prépare un gel douche nacré de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Huile 47 V 500000 vendue par la société RHONE POULENC | 2,5 g |
| - Composé de formule (I) dans laquelle : $R_1 = C_{16}H_{33}$ , $R_2 = C_{14}H_{29}$ $X = O$ , $Y = CH_2$ préparé par réaction de 3 moles d'alcool sur une mole d'époxyde , utilisé brut | 2,5 g |
| - Diéthanolamide de coprah | 2,5 g |
| - Copolymère de chlorure de diallyl diméthyl ammonium et d'acide acrylique vendu sous la dénomination MERQUAT 280 par la société CALGON à 35% de MA | 0,18 g MA |
| - Conservateur , parfum qs | |
| - pH spontané | 6,5 |
| - Eau qsp | 100 g |

EXEMPLE 6

On prépare un shampooing gel nacré de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Triméthylsillyl amodiméthicone vendue en émulsion cationique à 35% de MA sous la dénomination DC 929 par la société DOW CORNING | 2,75 g MA |
| - Composé de formule (I) dans laquelle : $R_1 = R_2 = C_{16}H_{33}$ X = Y = O | 2,5 g |
| - Monoisopropanolamide d'acide de coprah | 1,5 g |
| - Conservateur , parfum qs | |
| - pH spontané | 6,5 |
| - Eau qsp | 100 g |

EXEMPLE 7

On prépare un shampooing gel nacré de composition suivante :

- Alkyl($C_{12}$-$C_{14}$)éther sulfate de sodium oxyéthyléné à 2,2 moles d'oxyde d'éthylène vendu à 28% de MA — 16,8 g MA

- Cocoylbétaïne à 32% de MA — 2,56 g MA

- PDMS à fonction gamma-hydroxypropyle de formule

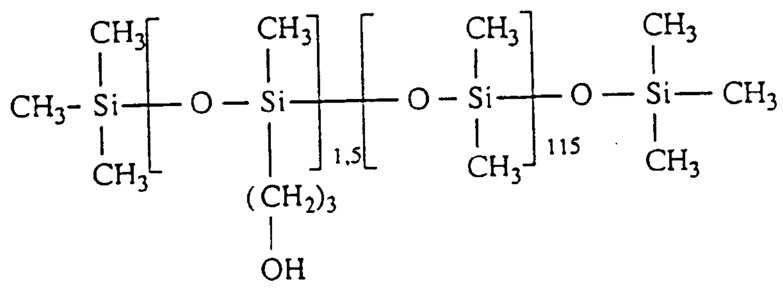

2,75 g

Mn = 9000

- Composé de formule (I) dans laquelle :
  $R_1 = C_{16}H_{33}$ , $R_2 = C_{14}H_{29}$
  X = O , Y = $CH_2$
  préparé par réaction de 3 moles d'alcool sur une mole d'époxyde , utilisé brut — 2,5 g

- Monoisopropanolamide d'acide de coprah — 0,5 g

- Conservateur , parfum qs

- pH spontané — 6,5

- Eau qsp — 100 g

<u>EXEMPLE 8</u>

On prépare un shampooing gel nacré de composition suivante :

| | |
|---|---|
| - Alkyl($C_{12}$-$C_{14}$)éther sulfate d'ammonium oxyéthyléné à 3 moles d'oxyde d'éthylène vendu à 25% de MA | 11,5 g MA |
| - Alkyl($C_{12}$-$C_{14}$) sulfate d'ammonium vendu à 30% de MA | 6 g MA |
| - Cocoylbétaïne à 32% de MA | 2,56 g MA |
| - Huile 47 V 500000 vendue par la société RHONE POULENC | 1 g |
| - Composé de formule (I) dans laquelle : $R_1 = C_{16}H_{33}$ , $R_2 = C_{14}H_{29}$ X = O , Y = $CH_2$ préparé par réaction de 3 moles d'alcool sur une mole d'époxyde , utilisé brut | 3 g |
| - Monoéthanolamide d'acide alkyl($C_{13}$-$C_{15}$)éthercarboxylique oxyéthyléné à 2 moles d'oxyde d'éthylène vendu sous la dénomination AMINOL A15 par la société CHEM Y | 1,5 g |
| - Gomme de guar hydroxypropylée et quaternisée vendue sous la dénomination JAGUAR C-13 S par la société MEYHALL | 0,25 g |
| -Conservateur , parfum qs | |
| -Eau qsp | 100 g |

EXEMPLE 9

On prépare un gel-douche de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Diméthiconecopolyol vendu sous la dénomination SILWETT L 722 par la société UNION CARBIDE | 10 g |
| - Composé de formule (I) dans laquelle : $R_1 = C_{16}H_{33}$ , $R_2 = C_{14}H_{29}$ $X = O$ , $Y = CH_2$ préparé par réaction de 3 moles d'alcool sur une mole d'époxyde , utilisé brut | 2,5 g |
| - Monoisopropanolamide d'acide de coprah | 1 g |
| - Conservateur , parfum qs | |
| - pH spontané | 6,3 |
| - Eau qsp | 100 g |

EXEMPLE 10

On prépare un shampooing de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Diméthiconecopolyol vendu sous la dénomination SILWETT L 722 par la société UNION CARBIDE | 3 g |
| - Composé de formule (I) dans laquelle : $R_1 = C_{16}H_{33}$ , $R_2 = C_{14}H_{29}$ $X = O$ , $Y = CH_2$ préparé par réaction de 3 moles d'alcool sur une mole d'époxyde , utilisé brut | 6 g |
| - Conservateur , parfum qs | |
| - pH spontané | 6,5 |
| - Eau qsp | 100 g |

EXEMPLE 11

On prépare un shampooing de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Diméthiconecopolyol vendu sous la dénomination SILWETT L 722 par la société UNION CARBIDE | 3 g |
| - Composé de formule (I) dans laquelle : $R_1 = C_{16}H_{33}$ , $R_2 = C_{14}H_{29}$ X = O , Y = $CH_2$ préparé par réaction de 3 moles d'alcool sur une mole d'époxyde , utilisé brut | 0,5 g |
| - Monoisopropanolamide d'acide de coprah | 1,5 g |
| - Alcool cétylique | 2 g |
| - Conservateur , parfum qs | |
| - pH spontané | 6,8 |
| - Eau qsp | 100 g |

EXEMPLE 12

On prépare un gel douche de composition suivante :

- Alkyl($C_{12}$-$C_{14}$)éther sulfate de sodium oxyéthyléné à 2,2 moles d'oxyde d'éthylène vendu à 28% de MA — 16,8 g MA

- Cocoylbétaïne à 32% de MA — 2,56 g MA

- PDMS à fonction gamma-hydroxypropyle de formule

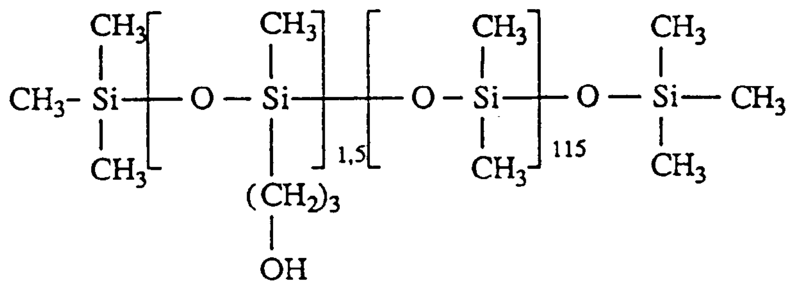

7,5 g

Mn ≈ 9000

- Composé de formule (I) dans laquelle :
$R_1 = C_{16}H_{33}$ , $R_2 = C_{14}H_{29}$
$X = O$ , $Y = CH_2$
préparé par réaction de 3 moles d'alcool sur une mole d'époxyde , utilisé brut — 2,5 g

- Monoisopropanolamide d'acide de coprah — 3 g

- Conservateur , parfum qs

- pH spontané — 7,4

- Eau qsp — 100 g

EXEMPLE 13

On prépare un gel-douche de composition suivante :

- Alkyl($C_{12}$-$C_{14}$)éther sulfate de sodium oxyéthyléné à 2,2 moles d'oxyde d'éthylène vendu à 28% de MA — 16,8 g MA

- Cocoylbétaïne à 32% de MA — 2,56 g MA

- Polydiméthyldiphénylsiloxane vendu sous la dénomination SILBIONE HUILE 70641V200 par la société RHONE POULENC — 10 g

- Composé de formule (I) dans laquelle :
  $R_1 = C_{16}H_{33}$ , $R_2 = C_{14}H_{29}$
  $X = O$ , $Y = CH_2$
  préparé par réaction de 3 moles d'alcool sur une mole d'époxyde , utilisé brut — 2,5 g

- Monoisopropanolamide d'acide de coprah — 3 g

- Conservateur , parfum qs

- pH spontané — 7,3

- Eau qsp — 100 g

EXEMPLE 14

On prépare un shampooing de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 16,8 g MA |
| - Cocoylbétaïne à 32% de MA | 2,56 g MA |
| - Polydiméthyldiphénylsiloxane vendu sous la dénomination SILBIONE HUILE 70641V200par la société RHONE POULENC | 3 g |
| - Composé de formule (I) dans laquelle : $R_1 = C_{16}H_{33}$ , $R_2 = C_{14}H_{29}$ X = O , Y = $CH_2$ préparé par réaction de 3 moles d'alcool sur une mole d'époxyde , utilisé brut | 6 g |
| - Monoisopropanolamide d'acide de coprah | 2 g |
| - Conservateur , parfum qs | |
| - pH spontané | 7,2 |
| - Eau qsp | 100 g |

EXEMPLE 15

On prépare un shampooing de composition suivante :

- Alkyl($C_{12}$-$C_{14}$)éther sulfate de sodium oxyéthyléné à 2,2 moles d'oxyde d'éthylène vendu à 28% de MA — 16,8 g MA

- Cocoylbétaïne à 32% de MA — 2,56 g MA

- PDMS à fonction gamma-hydroxypropyle de formule

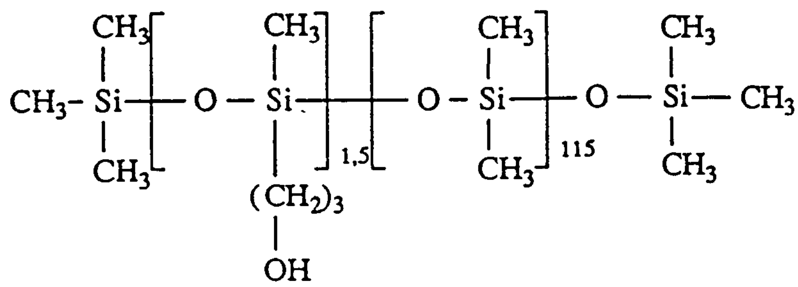

3 g

Mn ≈ 9000

- Composé de formule (I) dans laquelle :
  $R_1 = C_{16}H_{33}$ , $R_2 = C_{14}H_{29}$
  X = O , Y = $CH_2$
  préparé par réaction de 3 moles d'alcool sur une mole d'époxyde , utilisé brut — 0,5 g

- Monoisopropanolamide d'acide de coprah — 2 g

- Conservateur , parfum qs

- pH spontané — 7,1

- Eau qsp — 100 g

EXEMPLE 16

On prépare un shampooing de composition suivante :

| | |
|---|---|
| - Alkyl($C_{12}$-$C_{14}$)éther sulfate de sodium oxyéthyléné à 2,2 moles d'oxyde d'éthylène vendu à 28% de MA | 14 g MA |
| - Cocoylbétaïne à 32% de MA | 1,6 g MA |
| - Huile 47 V 500000 vendue par la société RHONE POULENC | 2 g |
| - Composé de formule (I) dans laquelle : $R_1 = R_2 = C_{12}H_{25}$ $X = S$ , $Y = CH_2$ | 2,5 g |
| - Diéthanolamide de coprah | 3 g |
| - Conservateur , parfum qs | |
| - pH spontané | 7 |
| - Eau qsp | 100 g |

## Revendications

**1.** Composition de lavage des matières kératiniques en particulier des cheveux et de la peau caractérisée par le fait qu'elle comprend dans un milieu aqueux au moins une silicone, au moins un agent tensio-actif possédant les propriétés détergentes et au moins un alcool ayant 27 à 44 atomes de carbone et comportant un ou deux groupements éther et/ou thioéther ou sulfoxyde répondant à la formule (I) :

$$R_1 - X\{C_2H_3(OH)\} CH_2 - Y - R_2 \qquad (I)$$

dans laquelle $R_1$ et $R_2$, identiques ou différents, désignent des groupements alkyle linéaires en $C_{12}$ à $C_{20}$;

X désigne un atome d'oxygène, un atome de soufre ou un groupement sulfoxyde;

Y désigne un atome d'oxygène, un atome de soufre, un groupement sulfoxyde ou un groupement méthylène;

dans le cas où Y désigne un groupement méthylène, la somme des nombres d'atomes de carbone de $R_1$ et $R_2$ varie de 24 à 40, et de préférence de 26 à 36 inclus, et lorsque Y ne désigne pas un groupement méthylène, la somme d'atomes de carbone de $R_1$ et $R_2$ varie de 24 à 40 inclus, et de préférence de 28 à 36 inclus; lorsque X ou Y désigne sulfoxyde, Y ou X ne désigne pas soufre.

**2.** Composition selon la revendication 1, caractérisée par le fait que l'alcool ayant 27 à 44 atomes de carbone répond à la formule (I) où X désigne oxygène, Y désigne méthylène et $R_1$ et $R_2$ désignent des radicaux ayant 12 à 18 atomes de carbone.

**3.** Composition selon la revendication 1, caractérisée par le fait que le composé de formule (I) est un composé répondant à la formule :

$$R_1 -X_1\{C_2H_3(OH)\}CH_2-Y_1-R_2 \qquad (IV)$$

dans laquelle $R_1$ et $R_2$ ont la même signification que dans la revendication 1;
$X_1$ désigne un atome d'oxygène ou un groupement sulfoxyde;
$Y_1$ désigne un atome d'oxygène, un groupement méthylène ou sulfoxyde, au moins un des symboles $X_1$ ou $Y_1$ représente un groupement sulfoxyde.

**4.** Composition selon l'une quelconque des revendications 1 à 3, caractérisée par le fait que les silicones sont choisies parmi les polyorganosiloxanes insolubles dans la composition et se présentent sous forme d'huiles, de cires, de résines ou de gommes.

**5.** Composition selon l'une quelconque des revendications 1 ou 4, caractérisée par le fait que les polyorganosiloxanes sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

**6.** Composition selon l'une quelconque des revendications 1 à 5, caractérisée par le fait que (a) les polyalkylsiloxanes sont choisis parmi :

- les polydiméthylsiloxanes linéaires à groupements terminaux triméthylsilyle
- les polydiméthylsiloxanes linéaires à groupements terminaux diméthylsilanol;
- les polyalkyl($C_1$-$C_{20}$)siloxanes;

(b) les polyalkylarylsiloxanes sont choisis parmi :

- les polydiméthylméthylphénylsiloxanes, les polydiméthyldiphényl siloxanes linéaires et/ou ramifiés; de viscosité comprise entre $1.10^{-5}$ et $5.10^{-2} m^2/s$ à 25°C;

(c) les gommes de silicone sont choisies parmi les polydiorganosiloxanes ayant des masses moléculaires comprises entre 200 000 et 1 000 000 utilisés seuls ou sous forme de mélange dans un solvant;
(d) les résines sont constituées d'unités :

$$R_2 \, Si \, O_{2/2}, \; R \, Si \, O_{3/2}, \; Si \, O_{4/2}$$

dans lesquelles R représente un groupement hydrocarboné ayant de 1 à 6 atomes de carbone ou un groupement phényle;
(e) les silicones organo modifiées sont choisies parmi les silicones comportant dans leur structure un ou plusieurs groupements organofonctionnels directement fixés sur la chaîne siloxanique ou fixés par l'intermédiaire d'un radical hydrocarboné.

**7.** Composition selon la revendication 6, caractérisée par le fait que les gommes de silicone utilisées seules ou sous forme de mélange sont choisies parmi les structures suivantes :

- polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxanes et les mélanges suivants :
- des mélanges formés à partir d'un polydiméthylsiloxane hydroxylé en bout de chaîne et d'un polydiméthylsiloxane cyclique;
- les mélanges formés à partir d'une gomme polydiméthylsiloxane et d'une silicone cyclique; et
- des mélanges de polydiméthylsiloxanes de viscosités différentes.

**8.** Composition selon la revendication 6, caractérisée par le fait que les silicones organo modifiées sont choisies par les polyorganosiloxanes comportant

a) des groupements polyéthylèneoxy et/ou polypropylène oxy;
b) des groupements aminés substitués ou non;
c) des groupements thiols;
d) des groupements carboxylates;
e) des groupements alcoxylés;
f) des groupements hydroxyalkyle répondant à la formule suivante :

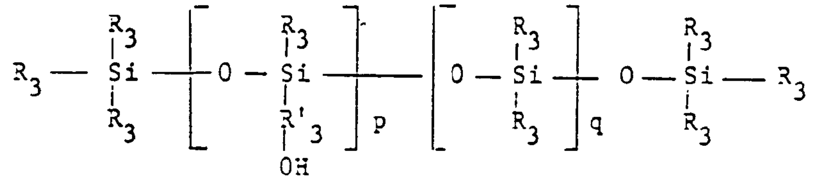 (V)

dans laquelle les radicaux $R_3$ identiques ou différents sont choisis parmi les radicaux méthyle et phényle, au moins 60% en mole des radicaux $R_3$ représentant méthyle; le radical $R'_3$ est un chaînon alkylène divalent hydrocarboné en $C_2$-$C_{18}$; p est compris entre 1 et 30 inclus; q est compris entre 1 et 150 inclus;

g) des groupements acyloxyalkyle répondant à la formule suivante :

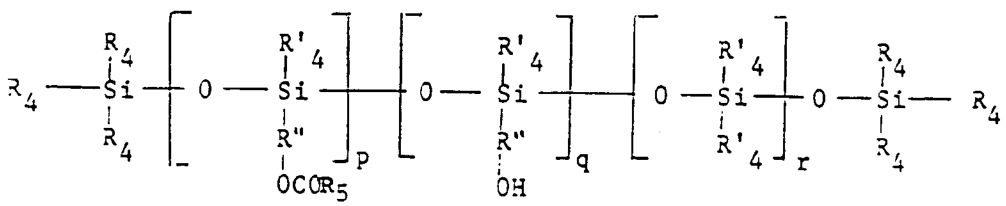 (VI)

dans laquelle :

$R_4$ désigne méthyle, phényle, $-OCOR_5$, hydroxyle, un seul des radicaux $R_4$ par atome de silicium pouvant être OH;

$R'_4$ désigne méthyle, phényle; au moins 60% en mole de l'ensemble des radicaux $R_4$ et $R'_4$ désignant méthyle;

$R_5$ désigne alkyle ou alcényle en $C_8$-$C_{20}$;

R" désigne un radical alkylène hydrocarboné divalent, linéaire ou ramifié en $C_2$-$C_{18}$;

r est compris entre 1 et 120 inclus;

p est compris entre 1 et 30;

q est égal à 0 ou est inférieur à 0,5 p, p+q étant compris entre 1 et 30; les polyorganosiloxanes de formule (VI) pouvant contenir des groupements

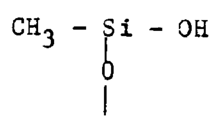

dans des proportions ne dépassant par 15% de la somme p+q+r;

h) des groupements alkyl carboxyliques,

i) des groupements 2-hydroxyalkylsulfonates,

j) des groupements 2-hydroxyalkylthiosulfates.

k) des groupements hydroxyacylamino.

**9.** Composition selon l'une quelconque des revendications 1 à 8, caractérisée par le fait que les polyorganosiloxanes sont choisis parmi les polyalkylsiloxanes linéaires à groupements terminaux triméthylsilylés, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

**10.** Composition selon l'une des revendications 1 à 4, caractérisée par le fait que les silicones sont choisies parmi les silicones volatiles.

**11.** Composition selon la revendication 10, caractérisée par le fait que que les silicones volatiles sont choisies parmi :

- les silicones cycliques comportant de 3 à 7 atomes de silicium;
- les cyclopolymères du type diméthylsiloxane/méthylalkylsiloxane de structure :

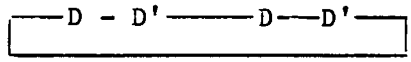

avec D = 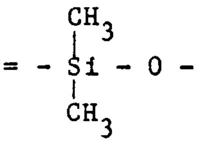 D' = 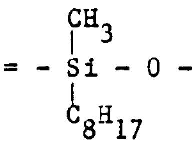

- les mélanges de silicones cycliques avec des composés organiques dérivés du silicium;
- les silicones volatiles linéaires ayant 2 à 9 atomes de silicium et de viscosité inférieure ou égale à $5.10^{-6}m^2/s$ à 25°C.

**12.** Composition selon l'une quelconque des revendications 1 à 11, caractérisée par le fait que les agents tensio-actifs détergents sont choisis parmi les agents tensio-actifs anioniques, amphotères, zwittérioniques, non ioniques ou leurs mélanges.

**13.** Composition selon la revendication 12, caractérisée par le fait que les agents tensio-actifs anioniques sont choisis parmi les sels alcalins, les sels de magnésium, les sels d'ammonium, les sels d'amines ou les sels d'aminoalcools des composés suivants :
- les alkylsulfates, alkyéther sulfates, alkylamidoéthersulfates, alkylarylpolyéthersulfates, monoglycérides sulfates;
- les alkylsulfonates, alkylamides sulfonates, alkylarylsulfonates, oléfines sulfonates, paraffines sulfonates;
- les alkylsulfosuccinates, les alkyléthersulfosuccinates, les alkylamides sulfosuccinates;
- les alkylsulfosuccinamates;
- les alkylsulfoacétates;
- les alkylphosphates, alkyléther phosphates;
- les acylsarcosinates, les acyliséthionates, N-acyltaurates; le radical alkyle ou acyle de ces différents composés étant constitué d'une chaîne carbonée comportant de 12 à 20 atomes de carbone,
- les sels d'acides gras avec des acides oléique, ricinoléïque, palmitique, stéarique;
- les acides d'huile de coprah, d'huile de coprah hydrogénée;
- les acyl lactylates dont le radical acyle comporte de 8 à 20 atomes de carbone,
- les acides éther carboxyliques polyoxyalkylénés répondant à la formule :

$$R_6\text{-}(OC_3H_6)_p\text{-}(OC_2H_4)_n\text{-}OCH_2COOA \qquad (VII)$$

dans laquelle $R_6$ désigne un radical ou un mélange de radicaux alkyle ou alcényle en $C_8$-$C_{22}$ linéaire ou ramifié, un radical alkyl($C_8$-$C_9$)-phényle, $R_7CONH\text{-}CH_2\text{-}CH_2$- avec $R_7$ désigne un radical alkyle ou alcényle linéaire ou ramifié en $C_{11}$-$C_{21}$; n est un nombre entier ou décimal compris entre 2 et 24; p est un nombre entier ou décimal compris entre 0 et 6; A désigne un atome d'hydrogène ou bien Na, K, Li, 1/2 Mg ou un reste monoéthanolamine, ammonium ou triéthanolamine.

**14.** Composition selon la revendication 12, caractérisée par le fait que les agents tensio-actifs non ioniques sont choisis parmi :

les alcools, les alkylphénols et acides gras polyéthoxylés, polyoxypropylénés ou polyglycérolés à chaîne grasse comportant 8 à 18 atomes de carbone, le nombre de groupements oxyde d'éthylène, oxyde de propylène étant compris entre 2 et 50 et le nombre de groupements glycérol étant compris entre 2 et 30; les copolymères oxyde d'éthylène et de propylène, les condensats d'oxydes d'éthylène et de propylène sur des alcools gras; les amides gras polyéthoxylés; les amines grasses polyéthoxylées; les éthanolamides d'acides gras; les esters d'acides gras du sorbitan oxyéthénés ou non; les esters d'acides gras du polyéthylèneglycol; les triesters phosphoriques; les esters d'acides gras du sucrose; des alkylpolyglycosides; les oxydes d'amines grasses;

**15.** Composition selon la revendication 12, caractérisée par le fait que l'agent tensio-actif non ionique est choisi parmi
- les composés de formule :

$$R_8O-(CH_2-\underset{\displaystyle CH_2OH}{CH}-O)_m H \qquad (VIII)$$

dans laquelle $R_8$ désigne un radical ou un mélange de radicaux alkyle comportant 10 à 14 atomes de carbone et m est un nombre statistique entier ou décimal de 2 à 10;

- les composés de formule :

$$R_9\text{-CONH-CH}_2\text{-CH}_2\text{-O-CH}_2\text{-CH}_2\text{-O(CH}_2\text{-CHOH-CH}_2\text{-O})_n H \qquad (IX)$$

dans laquelle $R_9$ désigne un radical ou un mélange de radicaux alkyle et/ou alcényle ayant de 11 à 17 atomes de carbone et n désigne un nombre statistique entier ou décimal de 1 à 5 et de préférence 1,5 à 4;

- les composés de formule :

$$R_{10}\text{-CHOH-CH}_2\text{-O-(CH}_2\text{-CHOH-CH}_2\text{-O})_p H \qquad (X)$$

dans laquelle $R_{10}$ désigne un radical aliphatique, cycloaliphatique, arylaliphatique ayant de préférence 7 à 21 atomes de carbone et leurs mélanges, les chaînes aliphatiques désignant en particulier des chaînes alkyle pouvant comporter de 1 à 6 groupements éther, thioéther et/ou hydroxyméthylène et p est compris entre 2 et 10;

- les composés préparés par condensation en catalyse acide entre 2 et 10 et de préférence 2,5 à 6 moles de glycidol par mole d'alcool ou d'alpha-diol contenant 10 à 14 atomes de carbone;
- des composés de poly(hydroxypropyléther) préparés par polyaddition de monochlorhydrine du glycérol sur un composé organique poly(hydroxylé) en présence d'une base forte.

**16.** Composition selon la revendication 12, caractérisée par le fait que les agents tensio-actifs amphotères ou zwittérioniques sont choisis parmi les dérivés d'amines secondaires ou tertiaires aliphatiques dans lesquels le radical aliphatique est une chaîne linéaire ou ramifiée comportant de 8 à 18 atomes de carbone et qui contient au moins un groupe anionique hydrosolubilisant, carboxylique, sulfonate, sulfate, phosphate, phosphonate;

- les alkylbétaïnes, les sulfobétaïnes, les amidobétaïnes ou les amidosulfobétaïnes.

**17.** Composition selon l'une quelconque des revendications 1 à 16, caractérisée par le fait que l'on utilise des mélanges de tensio-actifs choisis parmi les mélanges de tensio-actifs anioniques et d'agents tensio-actifs amphotères, zwittérioniques ou non ioniques.

**18.** Composition selon l'une quelconque des revendications 1 à 17, caractérisée par le fait que les silicones sont utilisées dans les compositions conformes à l'invention dans des proportions comprises entre 0,05 et 20% et de préférence entre 0,1 et 10% en poids par rapport au poids total de la composition.

**19.** Composition selon l'une quelconque des revendications 1 à 17, caractérisée par le fait que les alcools à groupements éther et/ou thioéther ou sulfoxyde de formule (I) ou (IV) sont utilisés dans des proportions suffisantes pour assurer l'homogénéité, l'épaississement et l'opalescence des compositions.

**20.** Composition selon la revendication 19 caractérisée par le fait que les alcools à groupements éther et/ou thioéther ou sulfoxyde de formule (I) ou (IV) sont présents dans des proportions comprises entre 0,1 et 10% en poids par rapport au poids total de la composition et en particulier entre 0,5 et 5%.

**21.** Composition selon l'une quelconque des revendications 1 à 19, caractérisée par le fait que les agents tensio-actifs sont présents dans une proportion suffisante pour conférer un caractère détergent à la composition.

**22.** Composition selon la revendication 21, caractérisée par le fait que les agents tensio-actifs sont présents dans des proportions comprise entre 5 et 50% en poids par rapport au poids total de la composition en particulier entre 8 et 35% en poids.

**23.** Composition selon l'une quelconque des revendications 1 à 22, caractérisée par le fait que le pH est compris entre 3 et 9 et en particulier entre 4 et 8.

**24.** Composition selon l'une quelconque des revendications 1 à 23, caractérisée par le fait que le milieu aqueux est constitué par de l'eau ou par un mélange d'eau et d'un solvant cosmétiquement acceptable choisi parmi les alcools inférieurs, les alkylèneglycols et les éthers de glycol.

**25.** Composition selon l'une quelconque des revendications 1 à 24, caractérisée par le fait que la composition contient en plus des agents régulateurs de viscosité choisis parmi les électrolytes, les hydrotropes, ou des agents épaississants présents en des proportions pouvant aller jusqu'à 10% en poids par rapport au poids total de la composition.

**26.** Composition selon l'une quelconque des revendications 1 à 25 caractérisée par le fait qu'elle contient en plus jusqu'à 3% d'agents nacrants et/ou opacifiants.

**27.** Composition selon l'une quelconque des revendications 1 à 26, caractérisée par le fait qu'elle contient en plus un ou plusieurs adjuvants destinés à améliorer les propriétés cosmétiques choisis par les tensio-actifs cationiques, les polymères anioniques ou non ioniques ou cationiques ou amphotères ou des protéines éventuellement quaternisées.

**28.** Composition selon la revendication 27 caractérisée par le fait que les polymères cationiques sont choisis parmi les polymères comportant des groupements aminés primaires, secondaires, tertiaires et/ou quaternaires faisant partie de la chaîne polymère ou directement reliés à celle-ci et ayant un poids moléculaire de 500 environ à 5 000 000 environ.

**29.** Composition selon la revendication 28, caractérisée par le fait que les polymères cationiques sont choisis parmi les polysaccharides cationiques, les polyammonium quaternaires.

**30.** Composition selon la revendication 27, caractérisée par le fait que le polymère amphotère est un copolymère de diallyl dialkylammonium et d'acide acrylique ou méthacrylique et/ou un polymère dérivé du chitosane.

**31.** Composition selon l'une quelconque des revendications 1 à 30, caractérisée par le fait qu'elle contient différents adjuvants cosmétiquement acceptables choisis parmi les parfums, les conservateurs, les séquestrants, les synergistes de mousses, les stabilisateurs de mousses, les agents acidifants ou alcalinisants.

**32.** Utilisation comme shampooing de la composition telle que définie dans l'une quelconque des revendications 1 à 31.

**33.** Utilisation comme gel douche de la composition telle que définie dans l'une quelconque des revendications 1 à 31.

**34.** Procédé de lavage et de conditionnement des matières kératiniques, caractérisé par le fait que l'on applique sur ces matières au moins une composition telle que définie dans l'une quelconque des revendications 1 à 31 et qu'après un temps de pose on rince à l'eau les matières traitées.

## Claims

**1.** Composition for washing keratinous materials, especially the hair and the skin, characterised in that it comprises, in an aqueous medium, at least one silicone, at least one surfactant possessing detergent properties and at least one alcohol having 27 to 44 carbon atoms and containing one or two ether and/or thioether or sulphoxide group(s), corresponding to the formula (I):

$$R_1 - X\{C_2H_3(OH)\}CH_2 - Y - R_2 \qquad (I)$$

in which $R_1$ and $R_2$, which may be identical or different, denote linear $C_{12}$ to $C_{20}$ alkyl groups;

X denotes an oxygen atom, a sulphur atom or a sulphoxide group;

Y denotes an oxygen atom, a sulphur atom, a sulphoxide group or a methylene group;

in the case where Y denotes a methylene group, the sum of the numbers of carbon atoms in $R_1$ and $R_2$ varies from 24 to 40, and preferably from 26 to 36, inclusive, and when Y does not denote a methylene group, the sum of carbon atoms in $R_1$ and $R_2$ varies from 24 to 40 inclusive, and preferably from 28 to 36 inclusive; when X or Y denotes sulphoxide, Y or X does not denote sulphur.

2. Composition according to Claim 1, characterised in that the alcohol having 27 to 44 carbon atoms corresponds to the formula (I) in which X denotes oxygen, Y denotes methylene and $R_1$ and $R_2$ denote radicals having 12 to 18 carbon atoms.

3. Composition according to Claim 1, characterised in that the compound of formula (I) is a compound corresponding to the formula:

$$R_1\text{-}X_1\text{+}C_2H_3(OH)\text{+}CH_2\text{-}Y_1\text{-}R_2 \qquad \text{(IV)}$$

in which $R_1$ and $R_2$ have the same meaning as in Claim 1;
$X_1$ denotes an oxygen atom or a sulphoxide group;
$Y_1$ denotes an oxygen atom or a methylene or sulphoxide group;
at least one of the symbols $X_1$ or $Y_1$ represents a sulphoxide group.

4. Composition according to any one of Claims 1 to 3, characterised in that the silicone is selected from polyorganosiloxanes which are insoluble in the composition and which take the form of oils, waxes, resins or gums.

5. Composition according to either of Claims 1 and 4, characterised in that the polyorganosiloxanes are nonvolatile polyorganosiloxanes selected from polyalkylsiloxanes, polyarylsiloxanes, polyalkylarylsiloxanes, silicone gums and resins and polyorganosiloxanes modified with organic functional groups, as well as mixtures thereof.

6. Composition according to any one of Claims 1 to 5, characterised in that (a) the polyalkylsiloxanes are selected from:
   - linear polydimethylsiloxanes containing terminal trimethylsilyl groups;
   - linear polydimethylsiloxanes containing terminal dimethylsilanol groups;
   - poly($C_1$-$C_{20}$ alkyl)siloxanes;
   - (b) the polyalkylarylsiloxanes are selected from:
   - linear and/or branched polydimethylmethylphenylsiloxanes and polydimethyldiphenylsiloxanes of viscosity between $1 \times 10^{-5}$ and $5 \times 10^{-2}$ $m^2/s$ at 25°C;

   (c) the silicone gums are selected from polydiorganosiloxanes having molecular masses of between 200,000 and 1,000,000, used alone or in the form of a mixture in a solvent;
   (d) the resins consist of units:

   $$R_2\ Si\ O_{2/2},\ R\ Si\ O_{3/2},\ Si\ O_{4/2}$$

   in which units R represents a hydrocarbon group having from 1 to 6 carbon atoms or a phenyl group;
   (e) the silicones modified with organic groups are selected from silicones containing in their structure one or more organic functional groups bound directly to the siloxane chain or bound via a hydrocarbon radical.

7. Composition according to Claim 6, characterised in that the silicone gums used alone or in the form of a mixture are selected from the following structures:
   - polydimethylsiloxane/methylvinylsiloxane,
   - polydimethylsiloxane/diphenylsiloxane,
   - polydimethylsiloxane/phenylmethylsiloxane,
   - polydimethylsiloxane/diphenylsiloxane/methylvinylsiloxanes,
     and the following mixtures:
   - mixtures formed from a polydimethylsiloxane hydroxylated at the end of the chain and a cyclic polydimethylsiloxane;
   - the mixtures formed from a polydimethylsiloxane gum and a cyclic silicone, and
   - mixtures of polydimethylsiloxanes of different viscosities.

8. Composition according to Claim 6, characterised in that the silicones modified with organic groups are selected from polyorganosiloxanes containing
   a) polyethylenoxy and/or polypropylenoxy groups;
   b) substituted or unsubstituted amino groups;
   c) thiol groups;
   d) carboxylate groups;
   e) alkoxy groups;
   f) hydroxyalkyl groups corresponding to the following formula:

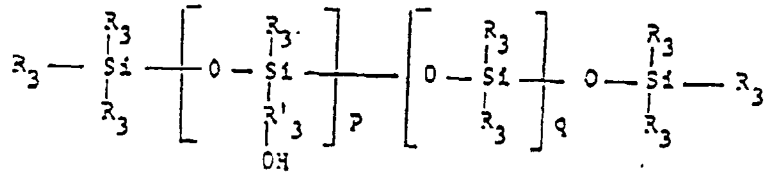 (V)

in which the radicals $R_3$, which may be identical or different, are selected from methyl and phenyl radicals, at least 60 mol% of the radicals $R_3$ denoting methyl; the radical $R'_3$ is a divalent $C_2$-$C_{18}$ carbon/hydrogen-based alkylene unit; p is between 1 and 30 inclusive; q is between 1 and 150 inclusive;
g) acyloxyalkyl groups corresponding to the following formula:

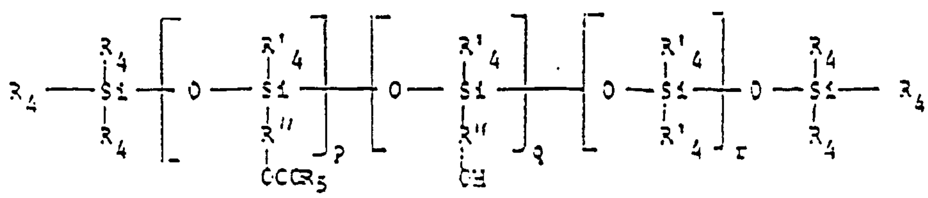 (VI)

in which:

$R_4$ denotes methyl, phenyl, $-OCOR_5$ or hydroxyl, it being possible for only one of the radicals $R_4$ per silicon atom to be OH;

$R'_4$ denotes methyl or phenyl; at least 60 mol% of all the radicals $R_4$ and $R'_4$ denoting methyl;

$R_5$ denotes $C_8$-$C_{20}$ alkyl or alkenyl;

R denotes a divalent, linear or branched $C_2$-$C_{15}$ carbon/hydrogen-based alkylene radical;

r is between 1 and 120 inclusive;

p is between 1 and 30;

q is equal to 0 or is less than 0.5 p, p + q being between 1 and 30; the polyorganosiloxanes of formula (VI) can contain groups

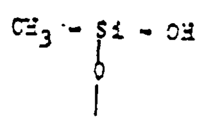

in proportions not exceeding 15% of the sum p + q + r;
h) alkylcarboxyl groups;
i) 2-hydroxyalkylsulphonate groups;
j) 2-hydroxyalkyl thiosulphate groups;
k) hydroxyacylamino groups.

**9.** Composition according to any one of Claims 1 to 8, characterised in that the polyorganosiloxanes are selected from linear polyalkylsiloxanes containing terminal trimethylsilyl groups, polyalkylarylsiloxanes, mixtures of two PDMS consisting of a gum and an oil of different viscosities, mixtures of organosiloxanes and cyclic silicones, and organopolysiloxane resins.

**10.** Composition according to one of Claims 1 to 4, characterised in that the silicones are selected from volatile silicones.

**11.** Composition according to Claim 10, characterised in that the volatile silicones are selected from:
- cyclic silicones containing from 3 to 7 silicon atoms;
- cyclic polymers of the dimethylsiloxane/methylalkylsiloxane type, of structure:

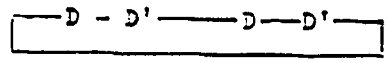

with D = 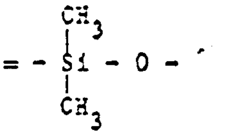 D' = 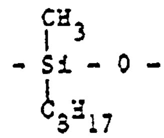

- mixtures of cyclic silicones with organic compounds derived from silicon;
- volatile linear silicones having 2 to 9 silicon atoms and of viscosity not exceeding 5 x $10^{-5}$ $m^2/s$ at 25°C.

**12.** Composition according to any one of Claims 1 to 11, characterised in that the detergent surfactants are selected from anionic, amphoteric, zwitterionic and nonionic surfactants or mixtures thereof.

**13.** Composition according to Claim 12, characterised in that the anionic surfactants are selected from the alkali metal salts, magnesium salts, ammonium salts, amine salts or amino alcohol salts of the following compounds:
- alkyl sulphates, alkyl ether sulphates, alkylamido ether sulphates, alkylaryl polyether sulphates, monoglyceride sulphates;
- alkylsulphonates, alkylamide sulphonates, alkylarylsulphonates, olefin sulphonates, paraffin sulphonates;
- alkylsulphosuccinates, alkyl ether sulphosuccinates, alkylamide sulphosuccinates;
- alkylsulphosuccinamates;
- alkylsulphoacetates;
- alkyl phosphates, alkyl ether phosphates;
- acylsarcosinates, acylisethionates, N-acyl-taurinates; the alkyl or acyl radical of these various compounds consisting of a carbon chain containing from 12 to 20 carbon atoms;
- the fatty acid salts with oleic, ricinoleic, palmitic and stearic acids;
- the acids of coconut oil and of hydrogenated coconut oil;
- the acyllactylates in which the acyl radical contains from 8 to 20 carbon atoms;
- the polyoxyalkylenated carboxylic ether-acids corresponding to the formula:

$$R_5\text{-}(OC_3H_6)_p\text{-}(OC_2H_4)_n\text{-}OCH_2COOA \qquad (VII)$$

in which $R_6$ denotes a linear or branched $C_8$-$C_{22}$ alkyl or alkenyl radical or a mixture of such radicals, a ($C_8$-$C_9$ alkyl)phenyl radical or $R_7CONH\text{-}CH_2\text{-}CH_2\text{-}$ with $R_7$ denoting a linear or branched $C_{11}$-$C_{21}$ alkyl or alkenyl radical; n is an integer or decimal number between 2 and 24, p is an integer or decimal number between 0 and 6; A denotes a hydrogen atom or alternatively Na, K, Li, 1/2 Mg or a monoethanolamine, ammonium or triethanolamine residue.

**14.** Composition according to Claim 12, characterised in that the nonionic surfactants are selected from:

polyethoxylated, polyoxypropylenated or polyglycerolated alcohols, alkylphenols and fatty acids having a fatty chain containing 8 to 18 carbon atoms, the number of ethylene oxide or propylene oxide groups being between 2 and 50 and the number of glycerol groups being between 2 and 30; copolymers of ethylene and propylene oxides, condensates of ethylene and propylene oxides with fatty alcohols; polyethoxylated fatty amides; polyethoxylated fatty amines; fatty acid ethanolamides; fatty acid esters of sorbitan, oxyethylenated or otherwise; fatty acid esters of polyethylene glycol; phosphoric triesters; fatty acid esters of sucrose; alkylpolyglycosides; and oxides of fatty amines.

**15.** Composition according to Claim 12, characterised in that the nonionic surfactant is selected from
- the compounds of formula:

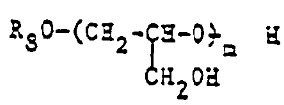 (VIII)

in which $R_8$ denotes an alkyl radical containing 10 to 14 carbon atoms or a mixture of such radicals

and m is an integral or decimal statistical value from 2 to 10;
- the compounds of formula:

$$R_9\text{-CONH-CH}_2\text{-CH}_2\text{-O-CH}_2\text{-CH}_2\text{-O}(\text{CH}_2\text{-CHOH-CH}_2\text{-O})_n\text{H} \qquad (IX)$$

in which $R_9$ denotes an alkyl and/or alkenyl radical having from 11 to 17 carbon atoms or a mixture of such radicals and n denotes an integral or decimal statistical value from 1 to 5, and preferably 1.5 to 4;
- the compounds of formula:

$$R_{10}\text{-CHOH-CH}_2\text{-O-}(\text{CH}_2\text{-CHOH-CH}_2\text{-O})_p\text{H} \qquad (X)$$

in which $R_{10}$ denotes an aliphatic, cycloaliphatic or arylaliphatic radical preferably having 7 to 21 carbon atoms and mixtures thereof, the aliphatic chains denoting, in particular, alkyl chains which can contain from 1 to 6 ether, thioether and/or hydroxymethylene groups and p is between 2 and 10;
- the compounds prepared by acid-catalysed condensation of between 2 and 10, and preferably 2.5 to 6, moles of glycidol per mole of alcohol or of alpha-diol containing 10 to 14 carbon atoms;
- poly(hydroxypropyl ether) compounds prepared by polyaddition of glycerol monochlorohydrin to a polyhydroxylated organic compound in the presence of a strong base.

**16.** Composition according to Claim 12, characterised in that the amphoteric or zwitterionic surfactants are selected from derivatives of secondary or tertiary aliphatic amines in which the aliphatic radical is a linear or branched chain containing from 8 to 18 carbon atoms and which contains at least one anionic carboxyl, sulphonate, sulphate, phosphate or phosphonate group conferring solubility in water;
- alkylbetaines, sulphobetaines, amidobetaines or amidosulphobetaines.

**17.** Composition according to any one of Claims 1 to 16, characterised in that mixtures of surfactants, selected from mixtures of anionic surfactants and amphoteric, zwitterionic or nonionic surfactants, are used.

**18.** Composition according to any one of Claims 1 to 17, characterised in that the silicones are used in the compositions according to the invention in proportions of between 0.05 and 20%, and preferably of between 0.1 and 10%, by weight relative to the total weight of the composition.

**19.** Composition according to any one of Claims 1 to 17, characterised in that the alcohols containing ether and/or thioether or sulphoxide groups of formula (I) or (IV) are used in sufficient proportions to provide for the homogeneity, thickening and opalescence of the compositions.

**20.** Composition according to Claim 19, characterised in that the alcohols containing ether and/or thioether or sulphoxide groups of formula (I) or (IV) are present in proportions of between 0.1 and 10% by weight relative to the total weight of the composition, and especially between 0.5 and 5%.

**21.** Composition according to any one of Claims 1 to 19, characterised in that the surfactants are present in a sufficient proportion to impart a detergent character to the composition.

**22.** Composition according to Claim 21, characterised in that the surfactants are present in proportions of between 5 and 50% by weight relative to the total weight of the composition, and especially between 8 and 35% by weight.

**23.** Composition according to any one of Claims 1 to 22, characterised in that the pH is between 3 and 9, and especially between 4 and 8.

**24.** Composition according to any one of Claims 1 to 23, characterised in that the aqueous medium consists of water or of a mixture of water and a cosmetically acceptable solvent selected from lower alcohols, alkylene glycols and glycol ethers.

25. Composition according to any one of Claims 1 to 24, characterised in that the composition contains, in addition, viscosity regulators selected from electrolytes, hydrotropes or thickening agents, present in proportions which can range up to 10% by weight relative to the total weight of the composition.

26. Composition according to any one of Claims 1 to 25, characterised in that it contains, in addition, up to 3% of pearlescence and/or opacifying agents.

27. Composition according to any one of Claims 1 to 26, characterised in that it contains, in addition, one or more adjuvants intended for improving the cosmetic properties, selected from cationic surfactants, anionic or nonionic or cationic or amphoteric polymers or optionally quaternised proteins.

28. Composition according to Claim 27, characterised in that the cationic polymers are selected from polymers containing primary, secondary, tertiary and/or quaternary amino groups forming part of the polymer chain or directly linked to the latter and having a molecular weight of approximately 500 to approximately 5,000,000.

29. Composition according to Claim 28, characterised in that the cationic polymers are selected from cationic polysaccharides and quaternary polyammonium compounds.

30. Composition according to Claim 27, characterised in that the amphoteric polymer is a copolymer of diallyldialkylammonium and acrylic or methacrylic acid and/or a polymer derived from chitosan.

31. Composition according to any one of Claims 1 to 30, characterised in that it contains various cosmetically acceptable adjuvants selected from fragrances, preservatives, sequestering agents, foam synergists, foam stabilisers and acidifying or alkalinising agents.

32. Use as a shampoo of the composition as defined in any one of Claims 1 to 31.

33. Use as a shower gel of the composition as defined in any one of Claims 1 to 31.

34. Process for washing and conditioning keratinous material, characterised in that at least one composition as defined in any one of Claims 1 to 31 is applied to the material , and in that, after an exposure time, the treated material is rinsed with water.

## Patentansprüche

1. Zusammensetzung zum Waschen keratinischer Stoffe, insbesondere der Haare und der Haut,
dadurch **gekennzeichnet,** daß
sie in einem wässrigen Medium enthält mindestens ein Silicon, ein oberflächenaktives Mittel mit Wascheigenschaften und mindestens einen Alkohol mit 27 bis 44 Kohlenstoffatomen und einer oder zwei Ether- und/oder Thioether- oder Sulfoxidgruppe(n) gemäß der Formel I
$$R_1 - X\{C_2H_3(OH)\}CH_2 - Y - R_2 \qquad (I)$$
worin $R_1$ und $R_2$, unabhängig voneinander, lineare $C_{12}$-$C_{20}$ -Alkylgruppen,
X ein Sauerstoffatom, Schwefelatom, eine Sulfoxidgruppe und
Y ein Sauerstoffatom, Schwefelatom, eine Sulfoxidgruppe oder Methylengruppe bedeuten,
mit der Maßgabe, daß,
wenn Y eine Methylengruppe darstellt, die Summe der Anzahl der in den Gruppen $R_1$ und $R_2$ vorhandenen Kohlenstoffatome einen Wert von 24 bis 40, vorzugsweise 26 bis 36, aufweist,
wenn Y eine Methylengruppe nicht darstellt, die Summe der Kohlenstoffatome von $R_1$ und $R_2$ einen Wert von 24 bis 40, vorzugsweise 28 bis 36, aufweist,
und wenn X oder Y eine Sulfoxidgruppe bedeuten, Y oder X nicht Schwefel darstellen.

2. Zusammensetzung gemäß Anspruch 1,
dadurch **gekennzeichnet,** daß
der Alkohol mit 27 bis 44 Kohlenstoffatomen der Formel (I) entspricht, worin X Sauerstoff, Y die Methylengruppe und $R_1$ und $R_2$ Reste mit 12 bis 18 Kohlenstoffatomen bedeuten.

3. Zusammensetzung gemäß Anspruch 1,

dadurch **gekennzeichnet,** daß
die Verbindung der Formel (I) eine Verbindung der Formel ist:

$$R_1\text{-}X_1\{C_2H_3(OH)\}CH_2\text{-}Y_1\text{-}R_2 \qquad \text{(IV)}$$

worin $R_1$ und $R_2$ dieselbe Bedeutung wie in Anspruch 1 haben,
$X_1$ ein Sauerstoffatom oder eine Sulfoxidgruppe,
$Y_1$ ein Sauerstoffatom, eine Methylen- oder Sulfoxidgruppe und
mindestens eines der Symbole $X_1$ oder $Y_1$ eine Sulfoxidgruppe darstellen.

**4.** Zusammensetzung gemäß jedem Anspruch 1 bis 3,
dadurch **gekennzeichnet,** daß
die Silicone aus in der Zusammensetzung unlöslichen Polyorganosiloxanen ausgewählt sind und in Form von Ölen, Wachsen, Harzen oder Gummiprodukten vorliegen.

**5.** Zusammenetzung gemäß jedem Anspruch 1 oder 4,
dadurch **gekennzeichnet,** daß
die Polyorganosiloxane nicht flüchtige Polyorganosiloxane sind, ausgewählt aus Polyalkylsiloxanen, Polyarylsiloxanen, Polyalkylarylsiloxanen, Gummiprodukten und Harzen von Siliconen, durch organofunktionelle Gruppen modifizierten Polyorganosiloxanen sowie deren Mischungen.

**6.** Zusammensetzung gemäß jedem Anspruch 1 bis 5,
dadurch **gekennzeichnet,** daß
(a) die Polyalkylsiloxane ausgewählt sind aus:
- linearen Polydimethylsiloxanen mit endständigen Trimethylsilylgruppen,
- linearen Polydimethylsiloxanen mit endständigen Dimethylsilanolgruppen,
- Poly-$C_{1-20}$- Alkylsiloxanen;

(b) die Polyalkylarylsiloxane ausgewählt sind unter:
- linearen und/oder verzweigten Polydimethylmethylphenylsiloxanen, Polydimethyldiphenylsiloxanen einer Viskosität von $1 \times 10^{-5}$ bis $5 \times 10^{-2}$ $m^2/s$ bei 25°C;

(c) die Gummiprodukte von Silicon ausgewählt sind aus Polydiorganosiloxanen mit Molekularmassen von 200000 bis 1 000 000, eingesetzt alleine oder in Form einer Mischung in einem Lösungsmittel;
(d) die Harze zusammengesetzt sind aus Einheiten:

$$R_2\,Si\,O_{2/2},\ R\,Si\,O_{3/2},\ Si\,O_{4/2}$$

worin R eine Kohlenwasserstoffgruppe mit 1 bis 6 Kohlenstoffatomen oder eine Phenylgruppe darstellt;
(e) die organisch modifizierten Silicone ausgewählt sind aus Siliconen, enthaltend in ihrer Struktur eine oder mehrere organofunktionelle Gruppen, die direkt an die Siloxankette oder über das Zwischenglied eines Kohlenwasserstoffrestes gebunden sind.

**7.** Zusammensetzung gemäß Anspruch 6,
dadurch **gekennzeichnet,** daß
die alleine oder in Form einer Mischung verwendeten Silicon-Gummiprodukte ausgewählt sind aus folgenden Strukturen:
- Polydimethylsiloxan/Methylvinylsiloxan,
- Polydimethylsiloxan/Diphenylsiloxan,
- Polydimethylsiloxan/Phenylmethylsiloxan,
- Polydimethylsiloxan/Diphenylsiloxan/Methylvinylsiloxan

sowie aus folgenden Mischungen:
- Mischungen aus einem am Kettenende hydroxilierten Polydimethylsiloxan und einem zyklischen Polydimethylsiloxan,
- Mischungen aus einem Polydimethylsiloxangummi und einem zyklischen Silicon sowie
- Mischungen von Polydimethylsiloxanen verschiedener Viskositäten.

**8.** Zusammensetzungen gemäß Anspruch 6,
dadurch **gekennzeichnet,** daß
die organisch modifizierten Silicone ausgewählt sind aus Polyorganosiloxanen, enthaltend
a) Polyethylenoxy- und/oder Polypropylenoxygruppen;
b) substituierte oder nicht substituierte Amingruppen;
c) Thiolgruppen;
d) Carboxylatgruppen;

e) Alkoxylgruppen;
f) Hydroxyalkylgruppen, die folgende Formel ergeben:

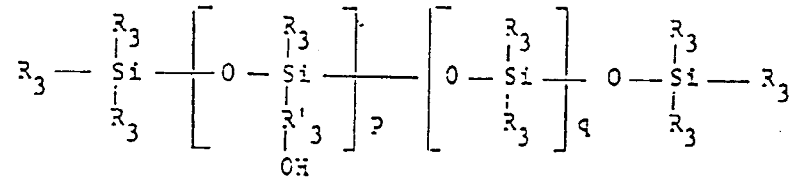 (V)

worin die Reste $R_3$, gleich oder verschieden, ausgewählt sind unter Methyl- und Phenylresten, wobei mindestens 60 Mol% der Reste $R_3$ Methyl bedeuten, der Rest $R'_3$ eine zweiwertige $C_{2-18}$-Kohlenwasserstoffalkylenkette, p 1 bis 30 und q 1 bis 150 sind;
g) Acyloxyalkylgruppen, die folgende Formel ergeben:

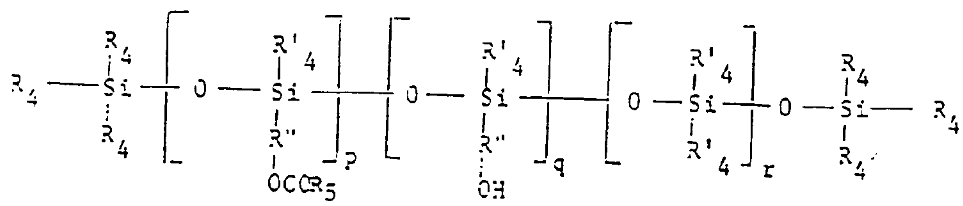 (VI)

worin:
$R_4$ eine Methyl-, Phenyl-, $-OCOR_5$-, Hydroxylgruppe, wobei ein einziger der Reste $R_4$ pro Siliziumatom OH sein kann;
$R'_4$ Methyl, Phenyl, wobei mindestens 60 Mol% aller Reste $R_4$ und $R'_4$ die Methylgruppe darstellen,
$R_5$ eine $C_{8-20}$ - Alkyl- oder -Alkenylgruppe,
R" einen linearen oder verzweigten zweiwertigen $C_{2-18}$- Kohlenwasserstoffalkylenrest bedeuten,
r 1 bis 120,
p 1 bis 30 betragen, und
q gleich 0 oder weniger als 0,5 p ist, wobei p+q 1 bis 30 beträgt, und worin die Polyorganosiloxane der Formel (VI) Gruppen:

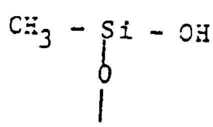

in Mengenverhältnissen enthalten können, die 15% der Summe p+q+r nicht überschreiten;
h) Alkylcarboxylgruppen;
i) 2-Hydroxyalkylsulfonatgruppen;
j) 2-Hydroxyalkylthiosulfatgruppen;
k) Hydroxyacylaminogruppen.

**9.** Zusammensetzung gemäß jedem Anspruch 1 bis 8, dadurch **gekennzeichnet,** daß die Polyorganosiloxane ausgewählt sind aus linearen Polyalkylsiloxanen mit endständigen Trimethylsilylgruppen, Polyalkylarylsiloxanen, Mischungen von 2 PDMS-Produkten aus einem Gummi und einem Öl mit verschiedenen Viskositäten, Mischungen aus Organosiloxanen und zyklischen Silikonen, Organopolysiloxanharzen.

**10.** Zusammensetzung gemäß einem der Ansprüche 1 bis 4, dadurch **gekennzeichnet,** daß die Silicone ausgewählt sind aus flüchtigen Siliconen.

**11.** Zusammensetzung gemäß Anspruch 10, dadurch **gekennzeichnet,** daß die flüchtigen Silicone ausgewählt sind aus:

- zyklischen Siliconen mit 3 bis 7 Siliziumatomen;
- Zyklopolymeren des Typs Dimethylsiloxan/Methylalkylsiloxan der Struktur:

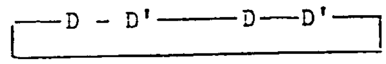

mit 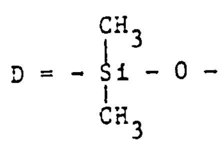 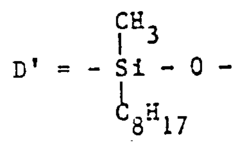

- Mischungen aus zyklischen Siliconen mit organischen Siliziumderivatverbindungen;
- flüchtigen linearen Siliconen mit 2 bis 9 Siliziumatomen und einer Viskosität unterhalb oder gleich 5 x $10^{-6}$ $m^2/s$ bei 25°C.

**12.** Zusammensetzung gemäß jedem Anspruch 1 bis 11, dadurch **gekennzeichnet,** daß die oberflächenaktiven Waschmittel ausgewählt sind aus anionischen, amphoteren, zwitterionischen, nicht ionischen oberflächenaktiven Mitteln und deren Mischungen.

**13.** Zusammensetzung gemäß Anspruch 12, dadurch **gekennzeichnet,** daß die anionischen oberflächenaktiven Mittel ausgewählt sind aus Alkali-, Magnesium-, Ammonium-, Amin- oder Aminoalkoholsalzen der folgenden Verbindungen:

- Alkylsulfaten, Alkylethersulfaten, Alkylamidoethersulfaten, Alkylarylpolyethersulfaten, Monoglyceridsulfaten;
- Alkylsulfonaten, Alkylamidsulfonaten, Alkylarylsulfonaten, Olefinsulfonaten, Paraffinsulfonaten;
- Alkylsulfosuccinaten, Alkylethersulfosuccinaten, Alkylamidsulfosuccinaten;
- Alkylsulfosuccinamaten;
- Alkylsulfoacetaten;
- Alkylphosphaten, Alkyletherphosphaten;
- Acylsarcosinaten, Acylisethionaten, N-Acyltauraten, wobei der Alkyl- oder Acylrest dieser verschiedenen Verbindungen aus einer Kohlenstoffkette mit 12 bis 20 Kohlenstoffatomen zusammengesetzt ist,
- Fettsäuresalzen mit Öl- , Rizinol-, Palmitin-, Stearinsäuren;
- Säuren von Kopraöl, hydriertem Kopraöl,
- Acyllactylaten, deren Acylrest 8 bis 20 Kohlenstoffatome enthält,
- Polyoxalkylierten Ethercarboxylsäuren der Formel:

$$R_6\text{-}(OC_3H_6)_p\text{-}(OC_2H_4)_n\text{-}OCH_2COOA \qquad \text{(VII)}$$

worin:

$R_6$ einen linearen oder verzweigten $C_{8_22}$-Alkyl- oder - Alkenylrest oder eine Mischung dieser Reste, $C_{8-9}$- Alkylphenylreste, $R_7CONH\text{-}CH_2CH_2$-Reste darstellt, worin $R_7$ einen linearen oder verzweigten $C_{11-21}$-Alkyl- oder - Alkenylrest bedeutet,

n eine ganze Zahl oder Dezimalzahl von 2 bis 24,

p eine ganze Zahl oder Dezimalzahl von 0 bis 6 und

A ein Wasserstoffatom oder auch Na, K, Li, 1/2 Mg oder einen Monoethanolamin-, Ammonium- oder Triethanolaminrest darstellen.

**14.** Zusammensetzung gemäß Anspruch 12, dadurch **gekennzeichnet,** daß die nicht ionischen oberflächenaktiven Mittel ausgewählt sind unter: polyethoxylierten, polyoxypropylierten oder polyglycerierten Alkoholen, Alkylphenolen und Fettsäuren mit

einer Fettkette mit 8 bis 18 Kohlenstoffatomen, wobei die Anzahl der Ethylen-, Propylenoxidgruppen 2 bis 50 und die Anzahl der Glyceringruppen 2 bis 30 betragen; aus Copolymeren von Ethylen- und Propylenoxid, Kondensaten von Ethylen- und Propylenoxiden mit Fettalkoholen; polyethoxylierten Fettamiden; polyethoxylierten Fettaminen; Fettsäureethanolamiden; oxethylierten oder nicht-ethoxylierten Fettsäureestern von Sorbitan; Fettsäureestern des Polyethylenglycols; Phosphorsäuretriestern; Fettsäureestern von Sucrose; Alkylpolyglycosiden; Fettaminoxiden.

**15.** Zusammensetzung gemäß Anspruch 12,
dadurch **gekennzeichnet,** daß das nicht ionische oberflächenaktive Mittel ausgewählt ist aus:
- Verbindungen der Formel:

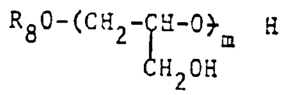 (VIII)

worin $R_8$ einen Alkylrest mit 10 bis 14 Kohlenstoffatomen oder eine Mischung dieser Reste und m eine statistische ganze Zahl oder Dezimalzahl von 2 bis 10 dartellen;
- Verbindungen der Formel:

$$R_9-CONH-CH_2-CH_2-O-CH_2-CH_2-O(CH_2-CHOH-CH_2-O)_n H \qquad (IX)$$

worin $R_9$ einen Alkyl- und/oder Alkenylrest mit 11 bis 17 Kohlenstoffatomen oder eine Mischung der Reste und n eine statistische ganze Zahl oder Dezimalzahl von 1 bis 5 und vorzugsweise von 1,5 bis 4 darstellen;
- Verbindungen der Formel:

$$R_{10}-CHOH-CH_2-O-(CH_2-CHOH-CH_2-O)_p H \qquad (X)$$

worin $R_{10}$ einen aliphatischen, cycloaliphatischen, araliphatischen Rest mit 7 bis 21 Kohlenstoffatomen und deren Mischungen darstellt, wobei die aliphatischen Ketten insbesondere Alkylketten bedeuten, die 1 bis 6 Ether-, Thioether- und/oder Hydroxymethylengruppen enthalten können, und p 2 bis 10 beträgt.;
- Verbindungen, hergestellt durch Kondensation unter saurer Katalyse von 2 bis 10 und vorzugsweise 2,5 bis 5 Mol Glycidol pro Mol Alkohol oder alpha-Diol mit 10 bis 14 Kohlenstoffatomen;
- Poly(hydroxypropylether)verbindungen, hergestellt durch Polyaddition des Monochlorhydrins von Glycerin mit einer poly-(hydroxylierten) organischen Verbindung in Gegenwart einer starken Base.

**16.** Zusammensetzung gemäß Anspruch 12,
dadurch **gekennzeichnet,** daß die amphoteren oder zwitterionischen oberflächenaktiven Mittel ausgewählt sind unter sekundären oder tertiären aliphatischen Aminderivaten, in denen der aliphatische Rest eine lineare oder verzweigte Kette mit 8 bis 18 Kohlenstoffatomen umfaßt und mindestens eine wasserlösliche anionische Carboxyl-, Sulfonat-, Sulfat-, Phosphat-, Phosphonatgruppe enthält;
- Alkylbetainen, Sulfobetainen, Amidobetainen oder Amidosulfobetainen.

**17.** Zusammensetzung gemäß jedem Anspruch 1 bis 16,
dadurch **gekennzeichnet,** daß man Mischungen oberflächenaktiver Mittel verwendet, ausgewählt aus Mischungen aus anionischen und aus amphoteren, zwitterionischen oder nicht ionischen oberflächenaktiven Mitteln.

**18.** Zusammensetzung gemäß jedem Anspruch 1 bis 17,
dadurch **gekennzeichnet,** daß die Silicone in den erfindungsgemäßen Zusammensetzungen im Mengenverhältnissen von 0,05 bis 20, vorzugsweise 0,1 bis 10, Gewichtsprozent, bezogen auf Gesamtgewicht der Zusammensetzung, verwendet werden.

**19.** Zusammensetzung gemäß jedem Anspruch 1 bis 17,
dadurch **gekennzeichnet,** daß die Alkohole mit Ether- und/oder Thioether- oder Sulfoxidgruppen der Formel (I) oder (IV) in zur Sicherstellung der Homogenität, Verdickung und Opaleszenz der Zusammensetzungen hinreichenden Mengenverhältnissen verwendet werden.

**20.** Zusammensetzung gemäß Anspruch 19,
dadurch **gekennzeichnet,** daß die Alkohole mit Ether- und/oder Thioether- oder Sulfoxidgruppen der Formel (I) oder (IV) in Mengenverhältnissen von 0,1 bis 10, insbesondere 0,5 bis 5, Gewichtsprozent vorliegen, bezogen auf Gesamtgewicht der Zusammensetzung.

**21.** Zusammensetzung gemäß jedem Anspruch 1 bis 19,
dadurch **gekennzeichnet,** daß die oberflächenaktiven Mittel in einem zur Übertragung von Waschvermögen auf die Zusammensetzung hinreichenden Mengenverhältnis vorliegen.

**22.** Zusammensetzung gemäß Anspruch 21,
dadurch **gekennzeichnet,** daß die oberflächenaktiven Mittel in Mengenverhältnissen von 5 bis 50, insbesondere 8 bis 35, Gewichtsprozent vorliegen, bezogen auf Gesamtgewicht der Zusammensetzung.

**23.** Zusammensetzung gemäß jedem Anspruch 1 bis 22,
dadurch **gekennzeichnet,** daß der pH 3 bis 9, insbesondere 4 bis 8, beträgt.

**24.** Zusammensetzung gemäß jedem Anspruch 1 bis 23,
dadurch **gekennzeichnet,** daß das wässrige Medium aus Wasser oder einer Mischung aus Wasser und einem kosmetisch verträglichen Lösungsmittel aus Niedrigalkoholen, Alkylenglycolen und Glycolethern zusammengesetzt ist.

**25.** Zusammensetzung gemäß jedem Anspruch 1 bis 24,
dadurch **gekennzeichnet,** daß die Zusammensetzung zusätzlich Viskositätsregulierungsmittel aus Elektrolyten, Hydrotropen oder Verdickungsmitteln enthält, welche in Mengenverhältnissen vorliegen, die bis zu 10 Gew.% ausmachen können, bezogen auf Gesamtgewicht der Zusammensetzung.

**26.** Zusammensetzung gemäß jedem Anspruch 1 bis 25,
dadurch **gekennzeichnet,** daß sie zusätzlich bis zu 3% an Mitteln enthalten, die einen Perlmuttglanz und/oder ein opakes Aussehen verleihen.

**27.** Zusammensetzung gemäß jedem Anspruch 1 bis 26,
dadurch **gekennzeichnet,** daß sie zusätzlich einen oder mehrere Hilfsstoffe zur Verbesserung der kosmetischen Eigenschaften enthält, ausgewählt aus kationischen oberflächenaktiven Mitteln, anionischen oder nicht ionischen oder kationischen oder amphoteren Polymeren oder gegebenenfalls quaternierten Proteinen.

**28.** Zusammensetzung gemäß Anspruch 27,
dadurch **gekennzeichnet,** daß die kationischen Polymeren ausgewählt sind aus Polymeren, enthaltend primäre, sekundäre, tertiäre und/oder quaternäre Amingruppen, welche einen Teil der Polymerkette bilden oder direkt an sie gebunden sind, mit einem Molekulargewicht von ca. 500 bis ca. 5 000 000.

**29.** Zusammensetzung gemäß Anspruch 28,
dadurch **gekennzeichnet,** daß die kationischen Polymeren ausgewählt sind aus kationischen Polysacchariden, quaternären Polyammoniumsalzen.

**30.** Zusammensetzung gemäß Anspruch 27,
dadurch **gekennzeichnet,** daß das amphotere Polymer ein Copolymer aus Diallyldialkylammoniumsalz und Acryl- oder Methacrylsäure und/oder ein von Chitosan abgeleitetes Polymer ist.

**31.** Zusammensetzung gemäß jedem Anspruch 1 bis 30,
dadurch **gekennzeichnet,** daß sie verschiedene kosmetisch verträgliche Hilfstoffe enthält, ausgewählt aus Parfüms, Konservierungsmitteln, Sequestriermitteln, Schaumsynergisten, Schaumstabilisierungsmitteln, sauer oder alkalisch machenden Mitteln.

**32.** Verwendung als Shampoo der gemäß jedem Anspruch 1 bis 31 definierten Zusammensetzung.

**33.** Verwendung als Duschgel der gemäß jedem Anspruch 1 bis 31 definierten Zusammensetzung.

**34.** Verfahren zum Waschen und Konditionieren keratinischer Materien, dadurch **gekennzeichnet,** daß man auf diese Materien mindestens eine in jedem Anspruch 1 bis 31 definierte Zusammensetzung aufbringt und nach einer Verweilzeit die behandelten Materien mit Wasser spült.